# EUROPEAN PATENT SPECIFICATION

(11) **EP 3 849 571 B1**
(45) Date of publication and mention of the grant of the patent: **04.09.2024**
(21) Application number: 19860431.6
(22) Date of filing: 10.09.2019
(51) Int. Cl.: A61K 39/00, C12N 5/0783, C12N 5/10, A61K 35/17, A61P 35/00

(54) **METHODS FOR EXPANDING ANTIGEN-SPECIFIC CAR-T CELLS, COMPOSITIONS AND USES RELATED THERETO**
VERFAHREN ZUR EXPANSION ANTIGENSPEZIFISCHER CAR-T-ZELLEN, ZUSAMMENSETZUNGEN UND VERWENDUNGEN IM ZUSAMMENHANG DAMIT
PROCÉDÉS D'EXPANSION DE CELLULES CAR-T SPÉCIFIQUES D'UN ANTIGÈNE, COMPOSITIONS ET UTILISATIONS ASSOCIÉES

(30) Priority: 10.09.2018 US 201862729089 P; 06.09.2019 US 201962896707 P
(43) Date of publication of application: 21.07.2021
(73) Proprietor: Atara Biotherapeutics, Inc., South San Francisco, CA 94080 (US)
(72) Inventor: AFTAB, Blake, T., San Francisco, CA 94080 (US); PHAM, Christina, San Francisco, CA 94080 (US); SHEN, Rhine, San Francisco, CA 94080 (US); WU, Michelle, San Francisco, CA 94080 (US)
(74) Representative: Vossius & Partner Patentanwälte Rechtsanwälte mbB
(86) International application number: PCT/US2019/050404
(87) International publication number: WO 2020/055862

(56) References cited:
- WO-A1-2016/154628
- WO-A1-2018/075813
- SHEN R R ET AL: "Abstract 2310: Functional demonstration of CD19 chimeric antigen receptor (CAR) engineered Epstein-Barr virus (EBV) specific T cells: An off-the-shelf, allogeneic CAR T-cell immunotherapy platform", CANCER RESEARCH, vol. 79, no. 13_Supplement, 2310, July 2019 (2019-07-01), AACR Annual Meeting; Atlanta, GA, USA; 29 March-3 April 2019, XP055951140, ISSN: 0008-5472, DOI: 10.1158/1538-7445.AM2019-2310
- SHEN R R ET AL: "CD19 chimeric antigen receptor (CAR) engineered epstein-barr virus (EBV) specific T cells - an off-the-shelf, allogeneic CAR T-cell immunotherapy platform", CYTOTHERAPY, vol. 21, no. 5, 8, May 2019 (2019-05-01), ICST 2019; Melboune, Australia; 29 May-1 June 2019, pages S11, XP055951142, ISSN: 1465-3249, DOI: 10.1016/j.jcyt.2019.03.569
- CHICAYBAM L ET AL: "CAR T Cells Generated Using Sleeping Beauty Transposon Vectors and Expanded with an EBV-Transformed Lymphoblastoid Cell Line Display Antitumor Activity In Vitro and In Vivo", HUMAN GENE THERAPY, vol. 30, no. 4, April 2019 (2019-04-01), pages 511 - 522, XP055951613, ISSN: 1043-0342, DOI: 10.1089/hum.2018.218
- C. R. Y. CRUZ, K. P. MICKLETHWAITE, B. SAVOLDO, C. A. RAMOS, S. LAM, S. KU, O. DIOUF, E. LIU, A. J. BARRETT, S. ITO, E. J. SHPALL,: "Infusion of donor-derived CD 19-redirected virus-specific T cells for B- cell malignancies relapsed after allogeneic stem cell transplant: a phase 1 study", BLOOD, vol. 122, no. 17, 12 September 2013 (2013-09-12), pages 2965 - 2973, XP055234516, ISSN: 0006-4971, DOI: 10.1182/blood-2013-06-506741
- MARTIN A PULE, BARBARA SAVOLDO, G DOUG MYERS, CLAUDIA ROSSIG, HEIDI V RUSSELL, GIANPIETRO DOTTI, M HELEN HULS, ENLI LIU, ADRIAN P : "Virus-specific T cells engineered to coexpress tumor-specific receptors: persistence and antitumor activity in individuals with neuroblastoma", NATURE MEDICINE, vol. 14, no. 11, 1 November 2008 (2008-11-01), pages 1264 - 1270, XP055021755, DOI: 10.1038/nm.1882
- C. ROSSIG, CATHERINE M BOLLARD, JED G NUCHTERN, CLIONA M ROONEY, MALCOLM K BRENNER: "Epstein- Barr virus-specific human T lymphocytes expressing antitumor chimeric T- cell receptors: potential for improved immunotherapy", BLOOD, vol. 99, no. 6, 15 March 2002 (2002-03-15), pages 2009 - 2016, XP055625826, ISSN: 0006-4971, DOI: 10.1182/blood.V99.6.2009
- ROSSIG C, PULE M, ALTVATER B, SAIAGH S, WRIGHT G, GHORASHIAN S, CLIFTON-HADLEY L, CHAMPION K, SATTAR Z, POPOVA B, HACKSHAW A, SMIT: "Vaccination to improve the persistence of CD 19CAR gene -modified T cells in relapsed pediatric acute lymphoblastic leukemia", LEUKEMIA, vol. 31, no. 5, 27 January 2017 (2017-01-27), pages 1087 - 1095, XP055794311, ISSN: 0887-6924, DOI: 10.1038/leu.2017.39
- JUNE, CARL H. : "Abstract PL03-01: CAR T cells for leukemia and more", CANCER RESEARCH , vol. 72, no. 8, 31 March 2012 (2012-03-31), pages PL03-01, XP009527046, Retrieved from the Internet <URL:https://cancerres.aacrjournals.org/content/72/8_Supplement/PL03-01.short>
- RUSHENI MUNISVARADASS, SURESH KUMAR, CHANDRAMOHAN GOVINDASAMY, KHALID ALNUMAIR, POOI MOK: "Human CD 3+ T-Cells with the Anti-ERBB2 Chimeric Antigen Receptor Exhibit Efficient Targeting and Induce Apoptosis in ERBB2 Overexpressing Breast Cancer Cells", INTERNATIONAL JOURNAL OF MOLECULAR SCIENCES, vol. 18, no. 9, 1797, 8 September 2017 (2017-09-08), pages 1 - 16, XP055693212

## Description

### BACKGROUND

Adoptive immunotherapy involves implanting or infusing disease-specific and/or engineered T cells, such as antigen-specific cytotoxic T cells (CTLs) and chimeric antigen receptor (CAR)-expressing T cells, into individuals with the aim of recognizing, targeting, and destroying disease-associated cells. Adoptive immunotherapies have become a promising approach for the treatment of many diseases and disorders, including cancer, post-transplant lymphoproliferative disorders, infectious diseases (e.g., viral infections), and autoimmune diseases.

For example, it has been shown that exposure to the ubiquitous Epstein Barr Virus (EBV, also known as human herpesvirus 4) can result in predisposition to or otherwise play a role in the pathogenesis of autoimmune diseases, including multiple sclerosis (MS), systemic autoimmune disease (SAD), and inflammatory bowel disease (IBD). Such pathologies (i.e., MS, SAD and IBD), arise from abnormal immune response against the body's own tissue. MS is characterized by the degradation of myelin, a protective lipid sheath surrounding nerve fibers, by the body's own immune cells. SADs are a group of connective tissue diseases with diverse symptoms that include rheumatoid arthritis (RA), systemic lupus erythematosus (SLE) and Sjögren's syndrome (SS). IBDs are a group of inflammatory conditions of the colon and small intestine that include Crohn's disease, celiac disease, and ulcerative colitis. Recent studies have shown that individuals diagnosed with MS show higher levels of EBV related proteins in B cells aggregated in nerve tissue than did healthy individuals.

The association between viral infection and the pathogenesis of multiple conditions *(e.g.,* cancers and autoimmune diseases) provided the impetus for several groups to develop the production of allogeneic (see, *e.g.,* WO 2017/203368 and autologous (see, *e.g.,* Pender et al., Multiple Sclerosis Journal. 2014, 20(11):1541-1544) antigen-specific T cells.

Immuno-surveillance by T cells plays a critical role in the detection and killing of a wide range of malignant cells (Gottschalk et al. 2005. Leuk Lymphoma. 46:1-10; Ochsenbein et al. 2002. Cancer Gene Ther. 9:1043-10). Survival and spread of malignant cells has been associated with the ability of such cells to evade recognition by CTLs (Bubenik et al. 2003. Oncol. Rep. 10:2005-2008; Rees et al. 1999. Cancer Immunol. Immunother. 48:374-381). In particular, antigen processing and presentation functions remain intact in the EBV-associated malignancies such as Hodgkin's lymphoma and nasopharyngeal carcinoma (Khanna et al. 1998. Cancer Res. 58: 310-314, Lee et al. 1998. Blood 92: 1020-1030). Recent studies have suggested that immuno-surveillance (and subsequent response) may be subverted by regulatory T cell-mediated suppression of the EBV-specific T cells, leading to failure to clear malignant cells. For example, the upregulation of inhibitory immune checkpoint receptors, such as PD-1 and Tim-3, correlates with T cell dysfunction and has been observed on both hepatitis C virus (HCV)-specific and HCV-nonspecific CD8⁺ T cells in patients with chronic HCV infection. Partial restoration of T cell proliferation and IFN-γ secretion can be achieved *ex vivo* by inhibiting the binding of PD-1 and Tim-3 to their respective ligands (i.e., B7-H1, also known as PD-L1, and Galectin-9). What is more, recent reports have demonstrated that prolonged administration of interferon therapy can lead to T cell "exhaustion", a dysfunctional state characterized by diminished proliferative potential, progressive loss of function, and sustained expression of inhibitory immune checkpoint receptors.

Chimeric antigen receptor (CAR)-T cells are an FDA-approved treatment for pediatric acute lymphoblastic leukemia and diffuse large cell lymphoma. The proliferation and persistence of CAR-T cells *in vivo* is an important factor in treatment efficacy. To this end, we herein describe methods for the generation and/or production of autologous and allogeneic antigen-specific T cells (e.g., T cells specifically sensitized to detect, for example, EBV-associated antigen(s)), and have also been engineered to express at least one functional chimeric antigen receptor directed against a disease-associated antigen of choice. The generation of antigen-specific CTLs that also express one or more CARs provides a novel adoptive immunotherapy platform for the treatment of a wide range of diseases. In addition, CAR-T cells representing central memory T cell (T_{cm}) and stem cell-like memory T cell (T_{scm}) phenotypes facilitate a proliferative potential and sustained *in vivo* expansion after CAR T-cell infusion (Kalos, 2018). Therefore, enriching adoptive immunotherapy products (e.g., CAR-T cells) for T_{cm} and T_{scm} phenotypes using enhanced culturing and stimulation methods is advantageous to the *in vivo* potency, persistence, and efficacy for this mode of therapy. Such augmentation of T cell responses against cancer and/or autoimmune-associated antigens, via the adoptive transfer of engineered antigen-specific T cells (e.g., antigen-specific, CAR-expressing T cells) may offer attractive alternatives to current treatment strategies.

### SUMMARY OF THE INVENTION

Provided herein are methods of generating allogeneic or autologous T cells that express a T cell receptor that specifically binds to an antigen *(e.g.,* a virus antigen such as an EBV peptide) presented on a class I major histocompatibility complex (MHC) and a chimeric antigen receptor (CAR) that specifically binds to a target-cell antigen or cell surface marker *(e.g.,* a cancer cell-associated antigen such as CD19). In some embodiments, the antigen-specific T cells are generated by incubating a sample comprising T cells (responder cells, *e.g.,* a PBMC sample or T cells isolated therefrom) with antigen presenting cells (APCs, i.e., stimulator cells) presenting an antigen (e.g., viral peptide) on a class I MHC *(e.g.,* a class I MHC encoded by an HLA allele that is present in the subject), thereby inducing proliferation of antigen-specific responder T cells. Preferably, the antigen-specific responder T cells are transduced with a viral vector comprising a nucleic acid sequence encoding a CAR. Also provided herein are methods for inducing *ex vivo* proliferation of a population of CAR-expressing, antigen-specific T cells, comprising culturing a population of isolated T cells with antigen-presenting stimulator cells and transducing the resultant antigen-specific T cells with a viral vector comprising a nucleic acid sequence encoding a CAR. In some embodiments, the transduced T cells are cultured with the antigen-presenting stimulator cells to induce proliferation of antigen-specific CAR T cells. In certain other embodiments, the isolated T cells are transduced with a CAR-encoding viral vector prior to culturing with APCs. In yet further embodiments, the isolated T cells are cultured with APCs prior to and following transduction with a CAR-encoding viral vector.

In some aspects, provided herein are *ex vivo* methods for enriching central memory T cells. In certain embodiments, such methods comprise obtaining a sample of cells from a subject comprising CD3⁺ T cells and contacting said CD3⁺ T cells with antigen-presenting stimulator cells. In preferred embodiments, the CD3⁺ T cells are isolated from the sample prior to contacting the antigen-presenting stimulator cells by methods known in the art *(e.g.,* positive selection of CD3⁺ cells from the sample and/or negative selection by depletion of undesired cells or components from the sample). For example, and without limitation, such methods include selection with anti-CD3 beads (*e.g.*, magnetic beads), plastic adherence, elutriation, depletion of NK cells (e.g., using anti-CD56 beads), and/or combinations thereof. In some such embodiments, the CD3⁺ T cells are transduced with a viral vector encoding a chimeric antigen receptor (CAR) before and/or after contact with the antigen-presenting stimulator cells. In some such embodiments the CAR-expressing, CD3⁺, antigen-specific T cells are cultured with antigen-presenting stimulator cells.

In some embodiments, the stimulator cells are made to present at least one virus peptide antigen by incubating the intended stimulator cells with native/wild type virus or with a viral vector encoding for the virus peptide antigen. In some such embodiments, the virus peptide antigens are derived from such types of viruses as herpesvirus (e.g., EBV and CMV), papillomavirus (e.g., HPV), adenovirus, polyomavirus (e.g., BKV, JCV, and Merkel cell virus), retrovirus (e.g., HTLV-I, also including lentivirus such as HIV), picornavirus (e.g., Hepatitis A virus), hepadnavirus (e.g., Hepatitis B virus), hepacivirus (e.g., Hepatitis C virus), deltavirus (e.g., Hepatitis D virus), hepevirus (e.g., Hepatitis E virus), oncovirus and the like. In certain preferred embodiments, the stimulator cells are made to present the EBV peptide by incubating PBMCs with native/wild type EBV. In other preferred embodiments, the stimulator cells are made to present the EBV peptide by incubating PBMCs with a viral vector encoding for the EBV peptide, thereby inducing the stimulator cells to present the EBV peptide. In some embodiments, the EBV peptide comprises a LMP1 peptide or a fragment thereof, a LMP2A peptide or fragment thereof, and/or an EBNA1 peptide or fragment thereof. In some embodiments, the EBV peptide comprises a sequence listed in Table 1. In some embodiments, the viral vector is a recombinant replication incompetent adenovirus (*e.g.*, AdE1-LMPpoly). In some embodiments, the stimulator cells may be B cells, antigen-presenting T cells, dendritic cells, or artificial antigen-presenting cells *(e.g.,* a cell line expressing CD80, CD83, 41BB-L and/or CD86, such as aK562 cells). Preferably, the antigen-presenting stimulator cells described herein also present a target-cell antigen or cell surface marker to the CAR (such as CD19). In some embodiments, the stimulator cells are irradiated.

Also provided herein are methods for identifying a therapeutic preparation of CAR-T cells as suitable for administration. In some embodiments, a sample of a therapeutic preparation of CAR-T cells is obtained and assessed for the abundance of multiple T cell types and subtypes. In some such embodiments, the amount of CD3⁺, CD4⁺, CD8⁺, CD62L⁺, and/or CD45RO⁺ cells are assessed.

In certain aspects, provided herein are methods for improving the proliferative capacity of T cells, comprising performing the methods disclosed herein. In some aspects, provided herein are methods for improving the viability of CAR-expressing T cells, comprising performing the methods disclosed herein. In certain aspects, provided herein are T cell compositions prepared by any one of the methods disclosed herein.

### BRIEF DESCRIPTION OF THE DRAWINGS

**Figure 1** shows a flow diagram outlining procedure and experimental groups comparing CAR transduction of EBV-T cells derived from PBMCs or isolated T cells.
**Figure 2** shows a sustained central memory phenotype in antigen-stimulated T cells (EBV-CTLs).
**Figure 3** shows growth curves of EBV-T cells derived from PBMCs and isolated T cells, and transduced to express a CAR.
**Figure 4** shows enrichment of viable CAR⁺ cells derived from isolated T cells following blasticidin selection.
**Figure 5** shows study design to examine the memory phenotype on T cells following stimulation and CAR transduction.
**Figure 6** shows representative flow cytometry gating strategy to identify CD3, CD4, CD8, CD62L, and CD45RO on CD19-CAR-T cells.
**Figure 7** shows representative dot plots for CD62L and CD45RO (gated on CD3⁺ cells) on CD19-CAR-T cells following no stimulation, BLCL, soluble anti-CD3/CD28, and bead-bound anti-CD3/CD28 stimulation. Naive PBMCs were also evaluated and served as a reference for gating memory subsets; red rectangle depicts CD62L expression on CD45RO⁺ cells.
**Figure 8** shows representative dot plots for CD4 and CD8 expression (gated on CD3) on CD19-CAR T cells.
**Figure 9** shows EBV-specific, anti-CD19 CAR T cells exert potent and specific cytotoxicity.
**Figure 10** shows EBV-specific, anti-CD19 CAR T cells induce CD19-specific cytotoxicity.
**Figure 11** shows specific and potent HLA-independnet cytolysis in both HLA-matched (BLCL targets) and HLA-mismatched (BLCL and Raji targets) cells, whereas EBV-CTLs could only induce significant cytolysis in matched BLCL target cells.
**Figure 12** shows conventionally generated anti-CD19 CAR T cells exhibiting enhanced alloreactive proliferation relative to EBV-specific, anti-CD19 CAR T cells.
**Figure 13** shows CellTrace^{™} Violet dilution assays upon co-culture with the indicated cell lines. Briefly, anti-CD19-CD28-CAR-EBV-CTLs induced cytolysis of CD19⁺ cell lines (i.e., BLCL, Raji, and NALM/6; bottom-left) while sparing autologous and allogeneic targets lacking CD19 and EBV antigen expression (i.e., K562 and PHA-blasts), with notably less alloreactivity relative to conventional CAR T cells (bottom-right, shaded).
**Figure 14** shows the that cytokine profile of EBV antigen-specific anti-CD19 CAR T cells and conventionally generated anti-CD 19 CAR T cells exhibit distinct responses.

### DETAILED DESCRIPTION

### General

The studies disclosed herein seek to determine the impact of various CAR-T cell stimuli amenable to a high-yield manufacturing process, and/or for enriching memory T cell immunophenotypes in a final therapeutic product. Standard anti-CD3/CD28 bead-based stimulation is widely used in the field as a method to expand T cells *ex vivo* or *in vitro* prior to transduction with CAR-encoding vectors. However, disclosed herein are manufacturing processes for antigen-stimulated T cells (e.g., virus-specific T cells) that also express one or more CARs. Such processes may comprise an initial T-cell enrichment step, wherein CD3⁺ T cells are enriched/purified from a more heterogeneous mixture of cells (e.g., from whole blood, from PBMCs, and the like); stimulated to recognize and respond to pre-selected antigens (e.g., viral antigens or other tumor/disease-associated antigens, etc.); andtransduced with one or more CAR constructs. For example, a sample of cells obtained from a subject comprising CD3⁺ T cells (e.g., PBMC) may be enriched for CD3⁺ T cells, and said CD3⁺ T cells brought into contact with antigen-presenting stimulator cells. Preferably, the CD3⁺ T cells are isolated from the sample prior to making contact with the antigen-presenting stimulator cells. More preferably, the T cells and antigen-presenting stimulator cells (e.g., BLCLs) are derived from the same sample and are thus HLA-matched. Such cell selection methods and techniques generally include positive selection of CD3⁺ and/or CD19⁺ cells from the sample and/or negative selection by depletion of undesired cells or components from the sample. For example, and without limitation, such methods comprise selection with live cell sorting techniques (e.g, fluorescence activated cell sorting), anti-CD3 and/or anti-CD19 beads (*e.g.*, magnetic beads), plastic adherence, depletion of NK cells, elutriation, and/or combinations thereof. The resultant CD3⁺ T cells may be transduced with a viral vector encoding a chimeric antigen receptor (CAR) before and/or after contact with antigen-presenting stimulator cells.

The study disclosed herein also provides a comparison of the central and effector memory T cell subsets of anti-CD19 CAR-T cells derived from stimulation of CTLs with either CD3/CD28 or BLCL co-cultures.

### Definitions

For convenience, certain terms employed in the specification, examples, and appended claims are collected here.

The articles *"a"* and *"an"* are used herein to refer to one or to more than one (*i.e.,* to at least one) of the grammatical object of the article. By way of example, "an element" means one element or more than one element.

As used herein, the term *"administering"* means providing a pharmaceutical agent or composition to a subject, and includes, but is not limited to, administering by a medical professional and self-administering. Such an agent can contain, for example, peptide described herein, an antigen presenting cell provided herein and/or a CTL provided herein.

The term *"binding"* or *"interacting"* refers to an association, which may be a stable association, between two molecules, *e.g.*, between a TCR and a peptide/MHC, due to, for example, electrostatic, hydrophobic, ionic and/or hydrogen-bond interactions under physiological conditions.

The term *"biological sample," "tissue sample,"* or simply *"sample"* each refers to a collection of cells obtained from a tissue of a subject. The source of the tissue sample may be solid tissue, as from a fresh, frozen and/or preserved organ, tissue sample, biopsy, or aspirate; blood or any blood constituents, serum, blood, bodily fluids such as cerebral spinal fluid, amniotic fluid, peritoneal fluid or interstitial fluid; or cells from any time in gestation or development of the subject.

As used herein, the term *"cytokine"* refers to any secreted polypeptide that affects the functions of cells and is a molecule which modulates interactions between cells in the immune, inflammatory or hematopoietic response. A cytokine includes, but is not limited to, monokines and lymphokines, regardless of which cells produce them. For instance, a monokine is generally referred to as being produced and secreted by a mononuclear cell, such as a macrophage and/or monocyte. Many other cells however also produce monokines, such as natural killer cells, fibroblasts, basophils, neutrophils, endothelial cells, brain astrocytes, bone marrow stromal cells, epidermal keratinocytes and B-lymphocytes. Lymphokines are generally referred to as being produced by lymphocyte cells. Examples of cytokines include, but are not limited to, Interleukin-1 (IL-1), Interleukin-2 (IL-2), Interleukin-6 (IL-6), Interleukin-7 (IL-7), Interleukin-8 (IL-8), Interleukin-15 (IL-15), Tumor Necrosis Factor-alpha (TNFα), and Tumor Necrosis Factor beta (TNFP).

The term "antibody fragment" refers to any derivative of an antibody which is less than full-length. In exemplary embodiments, the antibody fragment retains at least a significant portion of the full-length antibody's specific binding ability. Examples of antibody fragments include, but are not limited to, Fab, Fab', F(ab')2, scFv, Fv, dsFv diabody, Fc, and Fd fragments. The antibody fragment may be produced by any means. For instance, the antibody fragment may be enzymatically or chemically produced by fragmentation of an intact antibody, it may be recombinantly produced from a gene encoding the partial antibody sequence, or it may be wholly or partially synthetically produced. The antibody fragment may optionally be a single chain antibody fragment. Alternatively, the fragment may comprise multiple chains which are linked together, for instance, by disulfide linkages. The fragment may also optionally be a multimolecular complex. A functional antibody fragment will typically comprise at least about 50 amino acids and more typically will comprise at least about 200 amino acids.

The term "antigen binding site" refers to a region of an antibody that specifically binds an epitope on an antigen.

The term "single chain variable fragment" or "scFv" refers to an Fv fragment in which the heavy chain domain and the light chain domain are linked. One or more scFv fragments may be linked to other antibody fragments (such as the constant domain of a heavy chain or a light chain) to form antibody constructs having one or more antigen recognition sites.

The term "Fab fragment" refers to a fragment of an antibody comprising an antigen-binding site generated by cleavage of the antibody with the enzyme papain, which cuts at the hinge region N-terminally to the inter-H-chain disulfide bond and generates two Fab fragments from one antibody molecule.

The term "F(ab')2 fragment" refers to a fragment of an antibody containing two antigen-binding sites, generated by cleavage of the antibody molecule with the enzyme pepsin which cuts at the hinge region C-terminally to the inter-H-chain disulfide bond.

The term "Fc fragment" refers to the fragment of an antibody comprising the constant domain of its heavy chain.

The term "Fv fragment" refers to the fragment of an antibody comprising the variable domains of its heavy chain and light chain.

The term "engineered antibody" refers to a recombinant molecule that comprises at least an antibody fragment comprising an antigen binding site derived from the variable domain of the heavy chain and/or light chain of an antibody and may optionally comprise the entire or part of the variable and/or constant domains of an antibody from any of the Ig classes (for example IgA, IgD, IgE, IgG, IgM and IgY).

The term "specifically binds", "specific binding", or "targeting", as used herein, when referring to a polypeptide (including antibodies) or receptor, refers to a binding reaction which is determinative of the presence of the protein or polypeptide or receptor in a heterogeneous population of proteins and other biologics. Thus, under designated conditions (e.g. immunoassay conditions in the case of an antibody), a specified ligand or antibody "specifically binds" to its particular "target" (e.g., an antibody specifically binds to an endothelial antigen) when it does not bind in a significant amount to other proteins present in the sample or to other proteins to which the ligand or antibody may come in contact in an organism. Generally, a first molecule that "specifically binds" a second molecule has an affinity constant (Ka) greater than about 10⁵ M⁻¹ (e.g., 10⁶ M⁻¹, 10⁷ M⁻¹, 10⁸ M⁻¹, 10⁹ M⁻¹, 10¹⁰ M⁻¹, 10¹¹ M⁻¹, and 10¹² M⁻¹ or more) with that second molecule. For example, in the case of the ability of a TCR to bind to a peptide presented on an MHC (e.g., class I MHC or class II MHC); typically, a TCR specifically binds to its peptide/MHC with an affinity of at least a KD of about 10-4 M or less, and binds to the predetermined antigen/binding partner with an affinity (as expressed by KD) that is at least 10 fold less, at least 100 fold less or at least 1000 fold less than its affinity for binding to a non-specific and unrelated peptide/MHC complex (e.g., one comprising a BSA peptide or a casein peptide).

The term *"epitope"* means a protein determinant capable of specific binding to an antibody or TCR. Epitopes usually consist of chemically active surface groupings of molecules such as amino acids or sugar side chains. Certain epitopes can be defined by a particular sequence of amino acids to which an antibody is capable of binding

As used herein, the phrase *"pharmaceutically acceptable"* refers to those agents, compounds, materials, compositions, and/or dosage forms which are, within the scope of sound medical judgment, suitable for use in contact with the tissues of human beings and animals without excessive toxicity, irritation, allergic response, or other problem or complication, commensurate with a reasonable benefit/risk ratio.

As used herein, the phrase *"pharmaceutically-acceptable carrier"* means a pharmaceutically-acceptable material, composition or vehicle, such as a liquid or solid filler, diluent, excipient, or solvent encapsulating material, involved in carrying or transporting an agent from one organ, or portion of the body, to another organ, or portion of the body. Each carrier must be "acceptable" in the sense of being compatible with the other ingredients of the formulation and not injurious to the patient. Some examples of materials which can serve as pharmaceutically-acceptable carriers include: (1) sugars, such as lactose, glucose and sucrose; (2) starches, such as corn starch and potato starch; (3) cellulose, and its derivatives, such as sodium carboxymethyl cellulose, ethyl cellulose and cellulose acetate; (4) powdered tragacanth; (5) malt; (6) gelatin; (7) talc; (8) excipients, such as cocoa butter and suppository waxes; (9) oils, such as peanut oil, cottonseed oil, safflower oil, sesame oil, olive oil, corn oil and soybean oil; (10) glycols, such as propylene glycol; (11) polyols, such as glycerin, sorbitol, mannitol and polyethylene glycol; (12) esters, such as ethyl oleate and ethyl laurate; (13) agar; (14) buffering agents, such as magnesium hydroxide and aluminum hydroxide; (15) alginic acid; (16) pyrogen-free water; (17) isotonic saline; (18) Ringer's solution; (19) ethyl alcohol; (20) pH buffered solutions; (21) polyesters, polycarbonates and/or polyanhydrides; and (22) other non-toxic compatible substances employed in pharmaceutical formulations.

The terms *"polynucleotide",* and *"nucleic acid"* are used interchangeably. They refer to a polymeric form of nucleotides of any length, either deoxyribonucleotides or ribonucleotides, or analogs thereof. Polynucleotides may have any three-dimensional structure, and may perform any function. The following are non-limiting examples of polynucleotides: coding or non-coding regions of a gene or gene fragment, loci (locus) defined from linkage analysis, exons, introns, messenger RNA (mRNA), transfer RNA, ribosomal RNA, ribozymes, cDNA, recombinant polynucleotides, branched polynucleotides, plasmids, vectors, isolated DNA of any sequence, isolated RNA of any sequence, nucleic acid probes, and primers. A polynucleotide may comprise modified nucleotides, such as methylated nucleotides and nucleotide analogs. If present, modifications to the nucleotide structure may be imparted before or after assembly of the polymer. A polynucleotide may be further modified, such as by conjugation with a labeling component. In all nucleic acid sequences provided herein, U nucleotides are interchangeable with T nucleotides.

As used herein, a therapeutic that *"prevents"* a condition refers to a compound that, when administered to a statistical sample prior to the onset of the disorder or condition, reduces the occurrence of the disorder or condition in the treated sample relative to an untreated control sample, or delays the onset or reduces the severity of one or more symptoms of the disorder or condition relative to the untreated control sample.

As used herein, *"specific binding"* refers to the ability of a TCR to bind to a peptide presented on an MHC *(e.g.,* class I MHC or class II MHC). Typically, a TCR specifically binds to its peptide/MHC with an affinity of at least a K_{D} of about 10⁻⁴ M or less, and binds to the predetermined antigen/binding partner with an affinity (as expressed by K_{D}) that is at least 10 fold less, at least 100 fold less or at least 1000 fold less than its affinity for binding to a non-specific and unrelated peptide/MHC complex (e.g., one comprising a BSA peptide or a casein peptide).

As used herein, the term "*subject*" means a human or non-human animal selected for treatment or therapy.

In certain embodiments, agents of the invention may be used alone or conjointly administered with another type of therapeutic agent. As used herein, the phrase "conjoint administration" or "administered conjointly" refers to any form of administration of two or more different therapeutic agents (e.g., a composition comprising a CAR T disclosed herein and an inhibitor of an immune checkpoint) such that the second agent is administered while the previously administered therapeutic agent is still effective in the body (e.g., the two agents are simultaneously effective in the subject, which may include synergistic effects of the two agents). For example, the different therapeutic agents can be administered either in the same formulation or in separate formulations, either concomitantly or sequentially. In some preferred embodiments, the CAR T cells express (e.g., present on the cell surface or secrete) further therapeutic agents. In certain embodiments, the different therapeutic agents (e.g., CAR T cells and immune checkpoint-blocking molecules) can be administered within about one hour, about 12 hours, about 24 hours, about 36 hours, about 48 hours, about 72 hours, or about a week of one another. Thus, a subject who receives such treatment can benefit from a combined effect of different therapeutic agents.

As used herein, the term "treatment" refers to clinical intervention designed to alter the natural course of the individual being treated during the course of clinical pathology. Desirable effects of treatment include decreasing the rate of progression, ameliorating or palliating the pathological state, and remission or improved prognosis of a particular disease, disorder, or condition. An individual is successfully "treated," for example, if one or more symptoms associated with a particular disease, disorder, or condition are mitigated or eliminated.

The term *"vector"* refers to the means by which a nucleic acid can be propagated and/or transferred between organisms, cells, or cellular components. Vectors include plasmids, viruses, bacteriophage, pro-viruses, phagemids, transposons, and artificial chromosomes, and the like, that may or may not be able to replicate autonomously or integrate into a chromosome of a host cell.

### T cell enrichment

Until now, methods for the manufacture of CAR-T cells known in the art have relied on either a crude and heterogeneous starting material, such as PBMC (see Sun et al. Journal for ImmunoTherapy of Cancer (2015) 3:5) or highly purified isolates of specific T cell types, i.e., CD4⁺, CD8⁺ T cells, or specific ratios thereof (see Terakura et al. Blood (2011) 119:1 and Turtle et al. Sci Transl Med. (2016) Sep 7;8). However, the invention disclosed herein provides *ex vivo* methods for enriching antigen-specific T cells to be used in the manufacture of CAR-T cells. Notably, in some preferred embodiments, such methods comprise obtaining a sample of cells (e.g., PBMC) from a subject comprising CD3⁺ cells and contacting said CD3⁺ cells with antigen-presenting stimulator cells. Preferably, the CD3⁺ T cells are isolated from the sample prior to contacting the antigen-presenting stimulator cells by methods known in the art (*e.g*., positive selection of CD3⁺ cells from the sample and/or negative selection by depletion of undesired cells or components from the sample). For example, and without limitation, such methods include selection using fluorescence activated cell sorting (FACS), with anti-CD3 beads (*e.g.*, magnetic beads), plastic adherence, depletion of NK cells using anti-CD56, elutriation, and/or combinations thereof. Sensitizing the selected CD3⁺ cells to predetermined antigens, such as viral antigens, may promote a central memory phenotype in the resultant antigen-specific T cell population. Such T cells may be transduced with a viral vector encoding a chimeric antigen receptor (CAR) before and/or after contact with the antigen-presenting stimulator cells. In some such embodiments the CAR-expressing, CD3⁺, antigen-specific T cells are cultured with antigen-presenting stimulator cells.

The initial CD3⁺ enrichment step disclosed herein provides a starting material that is significantly less heterogeneous than a PBMC sample yet retains some level of heterogeneity over highly purified T cell fractions. Without wishing to be bound by theory, it is postulated that by using an initial CD3⁺ enrichment step, the starting material comprises a mixture of cells (including at least a plurality of effector T cell types, T helper cells (CD4⁺ T cells/ T_{H} cells), cytotoxic T cells (CD8⁺ T cells/ CTLs), memory T cell types (i.e., central memory T cells (T_{CM} cells), effector memory T cells (T_{EM} cells), tissue resident memory T cells (T_{RM}), and virtual memory T cells (T_{VM} cells)), regulatory T cells (T_{reg} cells), natural killer T cells (NKT cells), mucosal associated invariant cells (MAIT cells), gamma delta T cells (γδ T cells), double-negative T cells (DNTs), CD3⁺ B cells, or any combination thereof) which may work synergistically or provide an advantageous milieu to promote increased viability and proliferation of antigen-specific T cells following contact with APCs, improved transduction efficiency of CAR-expressing vector in such antigen-specific T cells, and a higher percentage of T_{CM} cells in the final therapeutic composition. In some embodiments, further enrichment is conducted following T cell stimulation with antigen. For example, NK depletion (e.g., CD56 depletion) may be employed prior to a subsequent antigen stimulation step (i.e. prior to re-stimulation of enriched, antigen-specific, T cells with antigen).

### Stimulation and Transduction

Manufacture of T cells expressing a T cell receptor that specifically binds to a peptide presented on a class I MHC requires T cell expansion against defined antigens. In some embodiments, the T cells also express a CAR that specifically binds to a disease-associated peptide *(e.g.,* a tumor-associate peptide, antigen, ligand, or the like).

Antigen delivery may be via viral infection by native virus, or via transduction using recombinant virus, of a sample of peripheral blood mononuclear cells (PBMCs) from healthy donors or isolated B cell lymphoblastoid cells (e.g., CD19⁺ BLCLs) therefrom. Thus the infected or transduced cells act as antigen-presenting cells and are referred to as "stimulators". In certain embodiments, the stimulator cells also express a peptide (i.e., antigen) on the cell surface that is recognized by the CAR. The stimulator cells may endogenously express such CAR-targeted peptides, or be engineered to express such peptides. The viral vector used to transduce stimulators may be a recombinant, replication incompetent virus (*e.g*., an adenovirus such as AdE1-LMPpoly). Alternatively, said stimulator cells are infected with a wild type/native virus. A separate sample or culture *(e.g.,* PBMCs from the same donor that are not used for transduction, a sample of PBMCs from a different healthy donor, or CD3⁺ cells isolated therefrom) comprises "responders" and contains T cells that become the active component of a therapy, expressing a T cell receptor that specifically binds to a peptide antigen presented on a class I MHC. The responder T cells may be isolated from PBMC by any suitable method, of which many are well known in the art. For example, "responder" T cells may be isolated from the sample prior to presentation to the stimulator fraction by methods known in the art such as anti-CD3 beads (*e.g.*, magnetic beads), plastic adherence, elutriation, and/or combinations thereof. Preferably, the "stimulator" cells (e.g., PBMCs or BLCLs) are obtained from the same cell population (e.g., PBMC sample) as are the "responder" cells such that they are identically HLA-matched. For example, a PBMC sample from a donor is split into a "stimulator" cell fraction and a "responder" cell fraction, wherein the stimulator cells may be enriched for CD3⁺ cells; and responder cells are transduced or infected so as to present particular antigens on the cell surface. Optionally, the stimulator cell fraction may be enriched by methods known in the art prior to transduction/infection, such as by selection for CD19⁺ cells. Thus, the antigen-presenting cells will present the antigen to T cells (e.g., CD3⁺-enriched cells), thus activating and inducing proliferation of antigen-specific T cells. In some such embodiments, the responder T cells *(e.g.,* isolated T cells) are transduced with a CAR-encoding vector prior to presentation of antigen by the stimulator fraction. In certain embodiments, the responder T cells (*e.g.*, isolated T cells) are transduced with a CAR-encoding vector after presentation of antigen by the stimulator fraction.

Accordingly, provided herein are methods of generating allogeneic or autologous T cells that express a T cell receptor that specifically binds to, for example, an EBV peptide presented on a class I MHC and further express a chimeric antigen receptor (CAR) that binds to a selected target *(e.g.,* CD19). In some embodiments, APCs are generated through viral infection of stimulator cells, *e.g.,* by wild type/native EBV or an adenoviral vector, such as AdE1-LMPpoly. The AdE1-LMPpoly vector encodes a polyepitope of defined CTL epitopes from LMP1 and LMP2 fused to a Gly-Ala repeat-depleted EBNA1 sequence. The AdE1-LMPpoly vector is described, for example, in Smith et al., Cancer Research 72:1116 (2012); Duraiswamy et al., Cancer Research 64:1483-9 (2004); and Smith et al., J. Immunol 117:4897-4906 (2006). In some embodiments, the stimulator cells are mixed with non-infected, isolated T cells (responders) so as to present the EBV polyepitopes to said T cells. In some embodiments, isolated, virus-specific T cells presented with EBV polyepitopes are activated and induced for proliferation.

T cells can be classified into naive or one of three major antigen-experienced subtypes: central memory T cell, effector memory T cell, and terminally differentiated effector T cells. Central memory T cells (T_{CM} cells) are commonly found in the lymph nodes and in the peripheral circulation. This subtype expresses CD45RO, C-C chemokine receptor type 7 (CCR7), and L-selectin (CD62L). Effector memory T cells (T_{EM} cells) lack lymph node-homing receptors and are thus found primarily in the peripheral circulation and tissues. T_{EM} cells express CD45RO but lack expression of CCR7 and L-selectin. In certain aspects, provided herein are methods for identifying a therapeutic preparation of CAR-T cells as suitable for administering to a recipient. In some embodiments, a sample of a therapeutic preparation of CAR-T cells is obtained and assessed for different T cell subtypes. Preferably, the preparation is comprised predominantly of CAR-expressing T_{cm} cells and/or CD4⁺ T cells.

In some embodiments, the responder cells and the stimulator cells are each derived from peripheral blood mononuclear cells (PBMC). In some such embodiments, the responder cells and the stimulator cells are each derived from PBMCs from the same donor. In other embodiments, the responder cells and the stimulator cells are each derived from PBMCs from different donors. In some such embodiments, prior to contact with stimulator cells, responder cells are isolated and/or purified so as to consist essentially of T cells. In preferred embodiments, the responder cells are isolated and/or purified so as to consist essentially of CD3⁺ cells. Most preferably, the responder cells consist essentially of CD3⁺ T cells.

Prior to presentation to responder cells *(e.g.,* T cells), stimulator cells may be infected with a native virus, such as EBV, thereby presenting viral antigens on their surface. In some embodiments, the stimulator cells are transduced with a viral vector, preferably an adenoviral vector comprising a nucleic acid sequence encoding a herpesvirus antigen. In some such embodiments, the adenoviral vector is replication incompetent. More preferably, the adenoviral vector comprises a nucleic acid sequence encoding one or more EBV antigens. The one or more EBV antigens may comprise an LMP1 peptide or fragment thereof, an LMP2A peptide or fragment thereof, and/or an EBNA1 peptide or fragment thereof. Most preferably, the adenoviral vector is AdE1-LMPpoly and encodes a polyepitope of defined CTL epitopes from LMP1 and LMP2 fused to a Gly-Ala repeat-depleted EBNA1 sequence. In some embodiments, the stimulator cells are incubated with one or more cytokines prior to culturing with (i.e., presentation to) responder cells *(e.g.,* non-infected PBMC or CD3⁺ enriched cells). Such stimulator cells may comprise B cells (e.g., BLCLs), antigen-presenting T-cells, dendritic cells, artificial antigen-presenting cells, and/or aK562 cells. In preferred embodiments, the stimulator cells are antigen-presenting BLCLs.

Though antigen-specific T cells achieve activation and proliferation when presented with antigen by the stimulator fraction, such stimulator cells are not desirable in the final harvested CAR-T cell product. Moreover, in order to minimize the risk of any viral recombination events in proliferating cells leading to formation of competent virus, the stimulator cells are treated and/or modified prior to culturing with responder T cells so as to inhibit proliferation, *e.g.*, by irradiation with gamma rays or exposure to an agent such as mitomycin C. For example, in such culture conditions, responder cells (e.g., T-cells) are presented with peptide antigens by non-proliferating stimulator cells. In some such embodiments, the culture is maintained from at least 24 hours to at least 28 days prior to transduction with a CAR-encoding vector. In some embodiments, the culture is maintained for at least 24 hours, at least 2 days, at least 3 days, at least 4 days, at least 5 days, at least 6 days, at least 7 days, at least 8 days, at least 9 days, at least 9 days, at least 10 days, at least 11 days, at least 12 days, at least 13 days, at least 14 days, at least 17 days, or at least 28 days prior to transduction with a CAR-encoding vector. Preferably, the culture is maintained for at least 2 days after antigen presentation by stimulator cells prior to transduction with a CAR-encoding vector. Most preferably, the culture is maintained for at least 6 days after antigen presentation by stimulator cells prior to transduction with a CAR-encoding vector. In further embodiments, the culture is maintained from at least 24 hours to at least 28 days following transduction with a CAR-encoding vector. In certain embodiments, the culture is maintained for at least 24 hours, at least 2 days, at least 3 days, at least 4 days, at least 5 days, at least 6 days, at least 7 days, at least 8 days, at least 9 days, at least 9 days, at least 10 days, at least 11 days, at least 12 days, at least 13 days, at least 14 days, at least 17 days, or at least 28 days following transduction with a CAR-encoding vector. In some embodiments, the culture is re-seeded and/or re-stimulated as necessary. For example, responder T cells undergo at least a first stimulation step (i.e., presented with antigen on APCs) and may be re-seeded and/or re-stimulated as needed (i.e., a second time or more). Said re-seeded and/or re-stimulated culture is maintained for at least 24 hours, at least 3 days, at least 9 days, at least 11 days, at least 14 days, at least 17 days, or at least 28 days prior to/ or following transduction with a CAR-encoding vector. In preferred embodiments, a responder cell culture is stimulated at least once prior to transduction with a CAR-encoding vector. More preferably, the responder culture is stimulated multiple times, wherein subsequent stimulation steps are separated by anywhere from 2 to 14 days. For example, a culture undergoing a first stimulation may undergo a second stimulation (e.g., re-stimulation) 11 days after the first stimulation; optionally, a third stimulation (e.g., re-stimulation) is initiated 7 days after the second stimulation step. A culture undergoing a stimulation step (e.g., re-stimulation) is maintained for at least 1 to 10 days before transduction with a CAR-encoding vector. Preferably, the culture is maintained for at least 2 days before transduction with a CAR-encoding vector. Most preferably, the culture is maintained for at least 6 days after antigen presentation by stimulator cells prior to transduction with a CAR-encoding vector. Optionally, an NK depletion step is employed prior to stimulation. For Example, CD56⁺ cells may be depleted from culture (e.g. with anti-CD56 beads) immediately prior to stimulation by APCs.

In certain embodiments, after at least a first presentation of antigen to achieve activation and proliferation, the antigen-specific T-cells may be frozen and stored prior to and/or following transduction with a CAR-encoding vector, to be thawed at a future date. For instance, the antigen-specific, CAR-expressing T cells contemplated herein may be administered to a subject in need thereof, or frozen for storage in a cell bank or repository to be thawed at a later date. In some such embodiments, the thawed culture is re-stimulated at least once more with antigen (e.g., with antigen-presenting stimulator cells, such as BLCLs and the like, as described herein) prior to and/or following transduction, as described herein. In some embodiments, said thawed culture is re-stimulated and/or re-seeded as necessary, and said re-stimulated and/or re-seeded culture is maintained for at least 24 hours, at least 2 days, at least 3 days, at least 9 days, at least 11 days, at least 14 days, at least 17 days, or at least 28 days prior to/ or following transduction with a CAR-encoding vector as described herein. Likewise, in such cases wherein the culture is re-stimulated multiple times, subsequent stimulation steps are separated by anywhere from 2 to 14 days, as described herein.

The activation and proliferation of antigen-specific T cells also requires a sufficient amount of antigen-presentation by stimulator cells. Accordingly, the stimulation cultures contemplated herein (e.g., including re-stimulation cultures) comprise known ratios of responder cells to simulator cells. For example, the ratio of responder cells to simulator cells is about 0.1:1 to about 20:1. Preferably, the ratio of responder cells to simulator cells is about 0.2:1 to about 5:1. In some embodiments, the ratio of responder cells to simulator cells is about 20:1, about 19: 1, about 18:1, about 17:1, about 16:1, about 15:1, about 14:1, about 13:1, about 12:1, about 11:1, about 10:1, about 9: 1, about 8:1, about 7:1, about 6:1, about 5:1, about 4: 1, about 3:1, about 2:1, about 1:1, about 0.9:1, about 0.8:1, about 0.7:1, about 0.6:1, about 0.5:1, about 0.4:1, about 0.3:1, about 0.2:1, or about 0.1:1. In some such embodiments, the ratio of responder cells to simulator cells is about 0.25:1, 0.43:1, or 4:1. Moreover, in such cases wherein the culture is re-stimulated multiple times, each stimulation may comprise the same ratio or a different ratio of responder cells to simulator cells. For example, and without limitation, in preferred embodiments, the initial stimulation comprises a responder: stimulator ratio of about 0.43:1. A subsequent stimulation (e.g., re-stimulation) may comprise a responder: stimulator ratio of 0.25:1 and a yet further stimulation (e.g., re-stimulation) comprises a responder: stimulator ratio of 4:1.

One of skill in the art would appreciate that cell proliferation in culture may vary and is limited by requirements for nutrients and oxygen, and by accumulation of waste products such as carbon dioxide and lactic acid. As such, one of skill in the art would be able to empirically determine appropriate culture and (if necessary) re-seeding schedules to achieve the T cells of the invention. In some embodiments, the culture is maintained until a predetermined harvesting date. Assessment of the culture and determination of the presence or absence of an active virus occurs prior to and/or on the day of harvest. Most preferably, the cultures and/or preparations disclosed herein are essentially free from active virus at the time of CTL harvest.

### Antigenic Peptides

In certain aspects, provided herein are methods of generating allogeneic or autologous CAR-T cells expressing TCRs that specifically bind to peptides (e.g., antigens) comprising T cell epitopes presented on class I MHC, and CARs that bind to a selected target, such as a disease-associated peptide target, for treating autoimmune disorders and cancers *(e.g.,* MS, SAD, IBD, and/or CD19⁺ B-cell malignancies, lymphomas, and leukemias). Contemplated herein are T cells suitable for the production of CAR-T cells generated by incubating a sample comprising T cells *(e.g.,* a PBMC sample or CD3⁺ cells isolated therefrom) with antigen-presenting cells (APCs) that present one or more of the T cell epitopes described herein *(e.g.,* APCs that present a peptide described herein comprising a EBV epitope on a class I MHC complex, such as EBV-infected or recombinantly transduced BLCLs).

In some such embodiments, the peptides comprising a T cell epitope, as described herein, comprise epitopes from viruses such as, and without limitation, herpesvirus (e.g., EBV and CMV), papillomavirus (e.g., HPV), adenovirus, polyomavirus (e.g., BKV, JCV, and Merkel cell virus), retrovirus (e.g., HTLV-I, also including lentivirus such as HIV), picornavirus (e.g., Hepatitis A virus), hepadnavirus (e.g., Hepatitis B virus), hepacivirus (e.g., Hepatitis C virus), deltavirus (e.g., Hepatitis D virus), and hepevirus (e.g., Hepatitis E virus), and the like. In some embodiments, the epitopes are HLA class I-restricted T cell epitopes. In other embodiments, the epitopes are HLA class II-restricted T cell epitopes.

The peptides provided herein may comprise a sequence of any EBV viral protein *(e.g.,* a sequence of at least 5, 6, 7, 8, 9, 10, 11, 12, 13, 14, 15, 16, 17, 18, 19 or 20 contiguous amino acids of any EBV protein). In some embodiments, the peptides provided herein comprise no more than 25, 20, 19, 18, 17, 16, 15, 14, 13, 12, 11 or 10 contiguous amino acids of the EBV viral protein.

The peptides provided herein may comprise a sequence of LMP1 *(e.g.,* a sequence of at least 5, 6, 7, 8, 9, 10, 11, 12, 13, 14, 15, 16, 17, 18, 19 or 20 contiguous amino acids of LMP1). In some embodiments, the peptides provided herein comprise no more than 25, 20, 19, 18, 17, 16, 15, 14, 13, 12, 11 or 10 contiguous amino acids of LMP1. An exemplary LMP1 amino acid sequence is provided below (SEQ ID NO: 1):

1     mdldlergpp gprrpprgpp lssyialall llllallfwl yiimsnwtgg
      allvlyafal
61    mlviiiliif ifrrdllcpl galcllllmi tlllialwnl hgqalylgiv
      lfifgcllvl
121   giwvyfleil wrlgatiwql lafflaffld illliialyl qqnwwtllvd
      llwlllflai
181   liwmyyhgqr hsdehhhdds lphpqqatdd ssnhsdsnsn egrhhllvsg
      agdapplcsq
241   nlgapgggpd ngpqdpdntd dngpqdpdnt ddngphdplp qdpdntddng
      pqdpdntddn
301   gphdplphnp sdsagndggp pnlteevenk ggdrgppsmt dggggdphlp
      tlllgtsgsg

361 gddddphgpv qlsyyd

In some embodiments, the peptides provided herein comprise a sequence of LMP2A (*e.g.*, a sequence of at least 5, 6, 7, 8, 9, 10, 11, 12, 13, 14, 15, 16, 17, 18, 19 or 20 contiguous amino acids of LMP2A). In some embodiments, the peptides provided herein comprise no more than 25, 20, 19, 18, 17, 16, 15, 14, 13, 12, 11 or 10 contiguous amino acids of LMP2A. An exemplary LMP2A amino acid sequence is provided below (SEQ ID NO: 2):

1     mgslemvpmg agppspggdp dgddggnnsq ypsasgsdgn tptppndeer
      esneeppppy
61    edldwgngdr hsdyqplgnq dpslylglqh dgndglpppp ysprddssqh
      iyeeagrgsm
121   npvclpviva pylfwlaaia ascftasvst vvtatglals llllaavass
      yaaaqrkllt
181   pvtvltavvt ffaicltwri edppfnsllf allaaagglq giyvlvmlvl
      lilayrrrwr
241   rltvcggimf lacvlvlivd avlqlspllg avtvvsmtll llafvlwlss
      pgglgtlgaa
301   lltlaaalal laslilgtln lttmfllmll wtlvvllics scsscpltki
      llarlflyal
361   allllasali aggsilqtnf kslsstefip nlfcmllliv agilfilail
      tewgsgnrty
421   gpvfmclggl ltmvagavwl tvmtntllsa wiltagflif ligfalfgvi
      rccryccyyc
481   ltleseerpp tpyrntv

In some embodiments, the peptides provided herein comprise a sequence of EBNA1 *(e.g.,* a sequence of at least 5, 6, 7, 8, 9, 10, 11, 12, 13, 14, 15, 16, 17, 18, 19 or 20 contiguous amino acids of EBNA1). In some embodiments, the peptides provided herein comprise no more than 25, 20, 19, 18, 17, 16, 15, 14, 13, 12, 11 or 10 contiguous amino acids of EBNA1. An exemplary EBNA1 amino acid sequence is provided below (SEQ ID NO: 3):

1 pffhpvgead yfeylqeggp dgepdvppga ieqgpaddpg egpstgprgq
gdggrrkkgg

61 wfgkhrgqgg snpkfeniae glrvllarsh vertteegtw vagvfvyggs
ktslynlrrg
121 talaipqcrl tplsrlpfgm apgpgpqpgp lresivcyfm vflqthifae
vlkdaikdlv
181 mtkpaptcni kvtvcsfddg vdlppwfppm vegaaaegdd gddgdeggdg
degeegqe

Preferably, the peptide comprises the sequence of an epitope listed in Table 1.

**Table 1: Exemplary EBV viral protein epitopes**

| **Epitope Sequence** | **HLA Restriction** | **SEQ ID NO.** |
|---|---|---|
| CLGGLLTMV | A*02 | 4 |
| FLYALALLL | A*02 | 5 |
| YLQQNWWTL | A*02, A*68, A*69 | 6 |
| YLLEMLWRL | A*02 | 7 |
| ALLVLYSFA | A*02 | 8 |
| LLSAWILTA | A*0203 | 9 |
| LTAGFLIFL | A*0206 | 10 |
| SSCSSCPLSKI | A*11 | 11 |
| PYLFWLAA | A*23, A*24, A*30 | 12 |
| TYGPVFMCL | A*24 | 13 |
| VMSNTLLSAW | A*25 | 14 |
| CPLSKILL | B*08 | 15 |
| RRRWRRLTV | B*27 | 16 |
| IEDPPFNSL | B*40 | 17 |
| IALYLQQNW | B*57, B*58 | 18 |
| MSNTLLSAW | B*58 | 19 |
| VLKDAIKDL | A*0203 | 20 |
| RPQKRPSCI | B*07 | 21 |
| IPQCRL TPL | B*07 | 22 |
| YNLRRGTAL | B*08 | 23 |
| HPVGEADYFEY | B*35 | 24 |
| LSRLPFGMA | B*57 | 25 |
| FVYGGSKTSL | Cw*03 | 26 |

In certain embodiments, the peptides provided herein comprise a sequence of any polyomavirus protein, e.g., peptides comprising BKV epitopes, JCV epitopes, MCV epitopes and/or epitopes that comprise sequences homologous between BKV, JCV and/or MCV epitopes that are recognized CTLs when presented on an HLA. In certain embodiments, the epitopes described herein are useful in the prevention and/or treatment of a polyomavirus infection (e.g., a BKV, JCV, or MCV viral infections) and/or cancer (e.g., a polyomavirus associated cancer expressing an epitope provided herein) and/or for the generation of pharmaceutical agents (e.g., CTLs and/or APCs) that are useful in the prevention and/or treatment of a polyomavirus infection (e.g., a BKV, JCV, or MCV viral infections) and/or cancer (e.g., a polyomavirus associated cancer expressing an epitope provided herein). In some embodiments, the epitope is a hybrid epitope comprising amino acids from both a BKV epitope and a homologous JCV epitope and/or amino acid variants found within different BKV or JCV epitopes. In some embodiments, the compositions and methods provided herein further comprise an MCV epitope. Exemplary peptides comprising BKV epitopes, JCV epitopes, MCV epitopes and/or epitopes that comprise sequences homologous between BKV, JCV and/or MCV epitopes (e.g., hybrid epitopes) can be found in WO 2018/049165.

In some embodiments, the peptides provided herein comprise a sequence of any cytomegalovirus protein, e.g., peptides comprising epitopes that are recognized by cytotoxic T lymphocytes (CTLs) and that are useful in the prevention and/or treatment of CMV infection and/or cancer (e.g., a cancer expressing a CMV epitope). Exemplary peptides comprising CMV epitopes can be found in each of WO2017203370, WO2014059489, and WO 2006/056027.

In some embodiments, the peptides provided herein comprise a sequence of any human papilloma virus protein, e.g., peptides comprising HPV epitopes that are recognized by cytotoxic T lymphocytes (CTLs) and that are useful in the prevention and/or treatment of HPV infection, and/or cancer (e.g., a cancer expressing an HPV epitope), and/or precancerous lesions. Exemplary peptides comprising HPV epitopes can be found in PCT/US2019/014727.

In some embodiments the peptides comprise epitopes from two or more viruses. In some embodiments the peptides comprise epitopes from three or more viruses. In some embodiments the peptides comprise epitopes from four or more viruses. In some embodiments the peptides comprise epitopes from five or more viruses. For example, in some embodiments the peptides comprise sequences from at least two, three, four or five of JCV, BKV, MCV, EBV, CMV and/or HPV.

In some embodiments, the peptides provided herein comprise two or more of the T cell epitopes (e.g., viral epitopes). In some embodiments, the peptides provided herein comprise at least 2, 3, 4, 5, 6, 7, 8, 9, 10, 11, 12, 13, 14, 15, 16, 17, 18, 19 or 20 T cell epitopes. For example, in some embodiments, the peptides provided herein comprise two or more of the T cell epitopes connected by linkers (e.g., polypeptide linkers). In some embodiments, the polypeptide or protein further comprises an intervening amino acid sequence between at least two of the plurality of epitopes. In some embodiments, the intervening amino acids or amino acid sequences are proteasome liberation amino acids or amino acid sequences. Non-limiting examples of proteasome liberation amino acids or amino acid sequences are or comprise AD, K or R. In some embodiments, the intervening amino acids or amino acid sequence are TAP recognition motifs. Typically, TAP recognition motifs may conform to the following formula: (R/N:I/Q:W/Y)n where n is any integer ≥ 1. Non-limiting examples of TAP recognition motifs include RIW, RQW, NIW and NQY. In some embodiments, the epitopes provided herein are linked or joined by the proteasome liberation amino acid sequence and, optionally, the TAP recognition motif at the carboxyl terminus of each epitope.

In some embodiments, the sequence of the peptides comprises a viral protein sequence except for 1 or more (e.g., 1, 2, 3, 4, 5, 6, 7, 8, 9, 10 or more) conservative sequence modifications. As used herein, the term "conservative sequence modifications" is intended to refer to amino acid modifications that do not significantly affect or alter the interaction between a T cell receptor (TCR) and a peptide containing the amino acid sequence presented on an MHC. Such conservative modifications include amino acid substitutions, additions (e.g., additions of amino acids to the N or C terminus of the peptide) and deletions (e.g., deletions of amino acids from the N or C terminus of the peptide). Conservative amino acid substitutions are ones in which the amino acid residue is replaced with an amino acid residue having a similar side chain. Families of amino acid residues having similar side chains have been defined in the art. These families include amino acids with basic side chains (e.g., lysine, arginine, histidine), acidic side chains (e.g., aspartic acid, glutamic acid), uncharged polar side chains (e.g., glycine, asparagine, glutamine, serine, threonine, tyrosine, cysteine, tryptophan), nonpolar side chains (e.g., alanine, valine, leucine, isoleucine, proline, phenylalanine, methionine), beta-branched side chains (e.g., threonine, valine, isoleucine) and aromatic side chains (e.g., tyrosine, phenylalanine, tryptophan, histidine). Thus, one or more amino acid residues of the peptides described herein can be replaced with other amino acid residues from the same side chain family and the altered peptide can be tested for retention of TCR binding using methods known in the art. Modifications can be introduced into an antibody by standard techniques known in the art, such as site-directed mutagenesis and PCR-mediated mutagenesis.

In some embodiments, the peptides provided herein comprise a sequence that is at least 80%, 85%, 90%, 95% or 100% identical to a protein sequence (e.g., the sequence of a fragment of a viral protein). To determine the percent identity of two amino acid sequences, the sequences are aligned for optimal comparison purposes (e.g., gaps can be introduced in one or both of a first and a second amino acid sequence for optimal alignment and non-identical sequences can be disregarded for comparison purposes). The amino acid residues at corresponding amino acid positions are then compared. When a position in the first sequence is occupied by the same amino acid residue as the corresponding position in the second sequence, then the molecules are identical at that position. The percent identity between the two sequences is a function of the number of identical positions shared by the sequences, taking into account the number of gaps, and the length of each gap, which need to be introduced for optimal alignment of the two sequences.

The peptide can be a chimeric or fusion peptide. As used herein, a "chimeric peptide" or "fusion peptide" comprises a peptide having a sequence provided herein linked to a distinct peptide having sequence to which it is not linked in nature. For example, the distinct peptide can be fused to the N-terminus or C-terminus of the peptide provided herein either directly, through a peptide bond, or indirectly through a chemical linker. In some embodiments, the peptide provided herein is linked to another peptide comprising a distinct epitope. In some embodiments, the peptide provided herein is linked to peptides comprising epitopes associated with other viral and/or infectious diseases. A chimeric or fusion peptide provided herein can be produced by standard recombinant DNA techniques. For example, DNA fragments coding for the different peptide sequences are ligated together in-frame in accordance with conventional techniques, for example by employing blunt-ended or stagger-ended termini for ligation, restriction enzyme digestion to provide for appropriate termini, filling-in of cohesive ends as appropriate, alkaline phosphatase treatment to avoid undesirable joining, and enzymatic ligation. In another embodiment, the fusion gene can be synthesized by conventional techniques including automated DNA synthesizers. Alternatively, PCR amplification of gene fragments can be carried out using anchor primers which give rise to complementary overhangs between two consecutive gene fragments which can subsequently be annealed and re-amplified to generate a chimeric gene sequence (see, for example, Current Protocols in Molecular Biology, Ausubel et al., eds., John Wiley & Sons: 1992). Moreover, many expression vectors are commercially available that already encode a fusion moiety.

The peptides provided herein can be isolated from cells or tissue sources by an appropriate purification scheme using standard protein purification techniques, and can be produced by recombinant DNA techniques, and/or can be chemically synthesized using standard peptide synthesis techniques. The peptides described herein can be produced in prokaryotic or eukaryotic host cells by expression of nucleotides encoding a peptide(s) of the present invention. Alternatively, such peptides can be synthesized by chemical methods. Methods for expression of heterologous peptides in recombinant hosts, chemical synthesis of peptides, and *in vitro* translation are well known in the art and are described further in Maniatis et al., Molecular Cloning: A Laboratory Manual (1989), 2nd Ed., Cold Spring Harbor, N. Y.; Berger and Kimmel, Methods in Enzymology, Volume 152, Guide to Molecular Cloning Techniques (1987), Academic Press, Inc., San Diego, Calif.; Merrifield, J. (1969) J. Am. Chem. Soc. 91:501; Chaiken I. M. (1981) CRC Crit. Rev. Biochem. 11:255; Kaiser et al. (1989) Science 243:187; Merrifield, B. (1986) Science 232:342; Kent, S. B. H. (1988) Annu. Rev. Biochem. 57:957; and Offord, R. E. (1980) Semisynthetic Proteins, Wiley Publishing.

In certain aspects, provided herein are nucleic acid molecules encoding the peptides described herein. In some embodiments, the nucleic acid molecule is a vector. In some embodiments, the nucleic acid molecule is a viral vector, such as an adenovirus based expression vector, that comprises the nucleic acid molecules described herein. In some embodiments, the vector provided herein encodes a plurality of epitopes provided herein *(e.g.,* as a polyepitope). In some embodiments, the vector provided herein encodes at least 2, 3, 4, 5, 6, 7, 8, 9, 10, 11, 12, 13, 14, 15, 16, 17, 18, 19 or 20 epitopes provided herein (*e.g*., epitopes provided in Table 1).

In some embodiments, the vector is a viral vector *(e.g.* an adenovirus, such as AdE1-LMPpoly). The AdE1-LMPpoly vector encodes a polyepitope of defined CTL epitopes from LMP1 and LMP2 fused to a Gly-Ala repeat-depleted EBNA1 sequence. The AdE1-LMPpoly vector is described, for example, in Smith et al., Cancer Research 72:1116 (2012); Duraiswamy et al., Cancer Research 64:1483-9 (2004); and Smith et al., J. Immunol 117:4897-4906 (2006).

As used herein, the term "vector," refers to a nucleic acid molecule capable of transporting another nucleic acid to which it has been linked. One type of vector is a "plasmid", which refers to a circular double-stranded DNA loop into which additional DNA segments may be ligated. Another type of vector is a viral vector, wherein additional DNA segments may be ligated into the viral genome. Certain vectors are capable of autonomous replication in a host cell into which they are introduced (*e.g.*, bacterial vectors having a bacterial origin of replication, episomal mammalian vectors). Other vectors (*e.g.*, non-episomal mammalian vectors) can be integrated into the genome of a host cell upon introduction into the host cell, and thereby be replicated along with the host genome. Moreover, certain vectors are capable of directing the expression of genes. Such vectors are referred to herein as "recombinant expression vectors" (or simply, "expression vectors"). In some embodiments, provided herein are nucleic acids operably linked to one or more regulatory sequences (*e.g.*, a promoter) in an expression vector. In some embodiments, the cell transcribes the nucleic acid provided herein and thereby expresses a peptide described herein. The nucleic acid molecule can be integrated into the genome of the cell or it can be extrachromasomal.

In some embodiments, provided herein are cells that contain a nucleic acid described herein *(e.g.,* a nucleic acid encoding a peptide described herein). The cell can be, for example, prokaryotic, eukaryotic, mammalian, avian, murine and/or human. In some embodiments, the cell is a mammalian cell. In some embodiments the cell is an APC *(e.g.* an antigen-presenting T cell, a dendritic cell, a B cell, or an aK562 cell). In the present methods, a nucleic acid described herein can be administered to the cell, for example, as nucleic acid without delivery vehicle, in combination with a delivery reagent. In some embodiments, any nucleic acid delivery method known in the art can be used in the methods described herein. Suitable delivery reagents include, but are not limited to, *e.g.,* the Mirus Transit TKO lipophilic reagent; lipofectin; lipofectamine; cellfectin; polycations (*e.g*., polylysine), atelocollagen, nanoplexes and liposomes. In some embodiments of the methods described herein, liposomes are used to deliver a nucleic acid to a cell or subject. Liposomes suitable for use in the methods described herein can be formed from standard vesicle-forming lipids, which generally include neutral or negatively charged phospholipids and a sterol, such as cholesterol. The selection of lipids is generally guided by consideration of factors such as the desired liposome size and half-life of the liposomes in the blood stream. A variety of methods are known for preparing liposomes, for example, as described in Szoka et al. (1980), Ann. Rev. Biophys. Bioeng. 9:467; and U.S. Pat. Nos. 4,235,871, 4,501,728, 4,837,028, and 5,019 369.

### CAR-T Cells

Provided herein are methods of treating cancers and autoimmune diseases (*e.g.*, MS, SAD, IBD) by administering to the subject allogeneic or autologous CAR-T cells expressing a T cell receptor that specifically binds to an antigen (e.g., a virus peptide antigen) presented on a or major histocompatibility complex (MHC) molecule, and a chimeric antigen receptor molecule with the ability to both specifically bind a selected target peptide and transduce subsequent activation signals via immunoreceptor activation motifs (ITAMs) present in their cytoplasmic tails.

In some embodiments, the antigen-specific T cells used for generating the CAR-T cells of the invention are selected from a cell bank or library. Preferably, the MHC is a class I MHC. In certain embodiments, the the MHC is a class II MHC and has an α chain polypeptide that is HLA-DMA, HLA-DOA, HLA-DPA, HLA-DQA or HLA-DRA. In some such embodiments, the class II MHC has a β chain polypeptide that is HLA-DMB, HLA-DOB, HLA-DPB, HLA-DQB or HLA-DRB. Such T cells, as described herein (e.g., antigen-specific T cells and antigen-specific T cells expressing a CAR), are stored in a cell library or bank before they are administered to the subject.

In some embodiments, the T cells used for generating the CAR-T cells of the invention are polyfunctional T-cells, i.e., those T cells that are capable of inducing multiple immune effector functions, that provide a more effective immune response to a pathogen than do cells that produce, for example, only a single immune effector *(e.g.* a single biomarker such as a cytokine or CD107a). Less-polyfunctional, monofunctional, or even "exhausted" T cells may dominate immune responses during chronic infections, thus negatively impacting protection against virus-associated complications. Likewise, the CAR-T cells of the invention are also polyfunctional (e.g., exhibit, retain, or have enhanced polyfunctionality). In some embodiments, at least 50% of the T cells used for generating the CAR-T cells of the invention are CD4⁺ T cells. In some such embodiments, said T cells are less than 50% CD4⁺ T cells. In still further embodiments, said T cells are predominantly CD4⁺ T cells. In some embodiments, at least 50% of the T cells used for generating the CAR-T cells of the invention are CD8⁺ T cells. In some such embodiments, said T cells are less than 50% CD8⁺ T cells. In still further embodiments, said T cells are predominantly CD8⁺ T cells.

Also provided herein are APCs that present a peptide described herein *(e.g.,* a peptide comprising a T cell epitope). In some embodiments the APCs are B cells, antigen presenting T-cells, dendritic cells, or artificial antigen-presenting cells *(e.g.,* aK562 cells). In certain preferred embodiments, the APCs are derived from lymphoblastoid cells, such as BLCLs. Exemplary antigen-presenting BLCLs are described in O'Reily et al., Immunol Res 2007 38:237-250 and Koehne et al., Blood 2002 99: 1730-1740.

Dendritic cells for use in the process may be prepared by taking PBMCs from a patient sample and adhering them to plastic. Generally, the monocyte population sticks and all other cells can be washed off. The adherent population is then differentiated with IL-4 and GM-CSF to produce monocyte derived dendritic cells. These cells may be matured by the addition of IL-1β, IL-6, PGE-1 and TNF-α (which upregulates the important co-stimulatory molecules on the surface of the dendritic cell) and are then transduced with one or more of the peptides provided herein.

In some embodiments, the APC is an artificial antigen-presenting cell, such as an aK562 cell. In some embodiments, the artificial antigen-presenting cells are engineered to express CD80, CD83, 41BB-L, and/or CD86. Exemplary artificial antigen-presenting cells, including aK562 cells, are described in U.S. Pat. Pub. No. 2003/0147869.

In certain aspects, provided herein are methods of generating APCs that present the one or more of the T cell epitopes described herein comprising contacting an APC with a peptide comprising an epitope and/or with a nucleic acid encoding said epitope. In some embodiments, the APCs are irradiated. A cell presenting a peptide described herein can be produced by standard techniques known in the art. For example, a cell may be pulsed to encourage peptide uptake. In some embodiments, the cells are transfected with a nucleic acid encoding a peptide provided herein. Provided herein are methods of producing antigen-presenting cells (APCs), comprising pulsing a cell with the peptides described herein. Exemplary examples of producing antigen presenting cells can be found in WO 2013/088114.

In some embodiments, provided herein are T cells (*e.g*., CD4 T cells and/or CD8 T cells) that express a TCR *(e.g.,* an αβ TCR or a γδ TCR) that recognizes a peptide described herein presented on a MHC. In some embodiments, the T cell is a CD8 T cell (a CTL) that expresses a TCR that recognizes a peptide described herein presented on a class I MHC. In some embodiments, the T cell is a CD4 T cell (a helper T cell) that recognizes a peptide described herein presented on a class II MHC.

In some embodiments, provided herein are methods of generating, activating and/or inducing proliferation of CAR-T cells that recognize one or more of the EBV epitopes described herein and also express a chimeric antigen receptor (*e.g.*, EBV-specific, antiCD19-CAR-T cells). In some embodiments, a culture (or sample thereof) comprising T cells (*i.e.,* isolated T cells) is incubated in culture with an APC provided herein *(e.g.,* an APC that presents a peptide comprising an EBV epitope on a class I MHC complex, such as the virally transduced PBMCs described herein). In some embodiments, the APCs are autologous to the subject from whom the T cells were obtained. In some embodiments, the APCs are not autologous to the subject from whom the T cells were obtained. In some embodiments, the sample containing T cells are incubated 2 or more times with APCs provided herein. In some embodiments, the T cells (and/or CAR-T cells) are incubated with the APCs in the presence of at least one cytokine. In some embodiments, the cytokine is IL-2, IL-4, IL-7 and/or IL-15. Similarly, T cell and/or CAR-T cell cultures may be maintained in the presence at least one cytokine, such as IL-2, IL-4, IL-7 and/or IL-15. Exemplary methods for inducing proliferation of T cells using APCs are provided, for example, in U.S. Pat. Pub. No. 2015/0017723. In preferred embodiments, such antigen-specific (e.g., EBV-specific) T cells are transduced with a vector comprising nucleic acid sequence encoding a chimeric antigen receptor (CAR) which generally comprise an extracellular domain (also referred to as an ectodomain), a transmembrane domain, and an intracellular signaling domain (also referred to as an endodomain).

The extracellular domain enables the CAR, when expressed on the surface of a T cell, to direct T cell activity to those cells expressing a target recognized by the extracellular domain. The inclusion of a costimulatory domain, such as the 4-1BB (CD137) costimulatory domain in series with CD3ζ in the intracellular region enables the T cell to receive co-stimulatory signals.

For the purpose of exemplification, first generation CARs represent an artificial receptor that, when expressed by T cells, can retarget them to a predetermined disease-associated antigen (e.g., tumor-associated antigens). Such CARs typically comprise a single chain variable fragment (scFv) derived from a target-specific antibody (e.g., a tumor antigen) fused to signaling domains from a T cell receptor (TCR), such as CD3ζ. Upon binding antigen, CARs trigger phosphorylation of immunoreceptor tyrosine-based activation motifs (ITAMS) and initiate the signal cascade required for cytolysis, cytokine secretion and proliferation, bypassing the endogenous antigen-processing pathway and MHC restriction. Second generation CAR designs include further signaling domains to enhance activation and co-stimulation, such as CD28 and/or 4-1BB. Second generation CARs are observed to induce more IL-2 secretion, increase T cell proliferation and persistence, mediate greater tumor rejection, and extend T cell survival. Third generation CARs are made by combining multiple signaling domains, such as CD3ζ-CD28-OC40 or CD3ζ-CD28-41BB, to augment potency with stronger cytokine production and killing ability.

The ectodomain of the CARs disclosed herein generally comprise a targeting domain, such as a ligand-binding domain or an antigen recognition domain that binds a target antigen. In certain embodiments, the targeting domain is from native T-cell receptor (TCR) alpha and beta single chains as have been described herein. Preferably, such targeting domains have simple ectodomains (e.g., a CD4 ectodomain to recognize HIV infected cells). Alternatively, such antigen recognition domains comprise exotic recognition components such as a linked cytokine (which leads can lead to recognition of cells bearing the cytokine receptor). Generally, with respect to the methods disclosed herein, almost anything that binds a given target with high affinity can be used as an antigen recognition region. Thus, such targeting domains are typically derived from an antibody. In some embodiments, the targeting domain is a functional antibody fragment or derivative thereof (e.g., an scFv or a Fab, or any suitable antigen-binding fragment of an antibody). In preferred embodiments, the ectodomain comprises a single-chain variable fragment (scFv). In some such embodiments, the scFv is from a monoclonal antibody (mAb). In preferred embodiments, the antigen-specific binding domain (e.g., the scFv) is fused to the transmembrane and/or signaling motifs involved in lymphocyte activation as disclosed in Sadelain M, et al. Nat Rev Cancer 2003 3:35-45, incorporated herein by reference in its entirety. It also optionally contains a signal peptide (SP) so that the CAR can be glycosylated and anchored in the cell membrane of the immune effector cell. The affinity/specificity of the scFv is driven in large part by specific sequences within complementarity determining regions (CDRs) in the heavy (V_{H}) and light (V_{L}) chain. Each V_{H} and V_{L} sequence will have three CDRs (CDR1, CDR2, CDR3).

In some embodiments, the targeting domain is derived from natural antibodies, such as monoclonal antibodies. In some cases, the antibody is human. In some cases, the antibody has undergone an alteration to render it less immunogenic when administered to humans. For example, the alteration may comprise one or more techniques selected from chimerization, humanization, CDR-grafting, deimmunization, and mutation of framework amino acids to correspond to the closest human germline sequence. In yet further embodiments, the targeting domain is a ligand-based targeting domain, wherein, for example, the scFv disclosed herein is replaced with a ligand for a tumor marker. Thus, a CAR that expresses a ligand for the IL 13 receptor (IL13R) would allow redirection of T cells to IL13R expressed on glioblastoma.

Also disclosed are bi-specific CARs that are capable of binding to at least two molecular targets (e.g., cell-specific markers and tumor antigens). Also disclosed are CARs designed to work only in conjunction with another CAR that binds a different antigen, such as a cancer-associated antigen. For example, in these embodiments, the endodomain of the first CAR contains only a signaling domain (SD) or a co-stimulatory signaling region (CSR), but not both. The second CAR provides the missing signal if it is activated. For example, if the disclosed CAR contains an SD but not a CSR, then the immune effector cell containing this CAR is only activated if another CAR (or T-cell) containing a CSR binds its respective antigen. Likewise, if the disclosed CAR contains a CSR but not a SD, then the immune effector cell containing this CAR is only activated if another CAR (or T-cell) containing an SD binds its respective antigen.

Exemplary antigens include, but are not limited to, tumor antigens, i.e., proteins that are produced by tumor cells that elicit an immune response, particularly T-cell mediated immune responses. The additional antigen binding domain can be an antibody or a natural ligand of the tumor antigen. The selection of the additional antigen binding domain will depend on the particular type of cancer to be treated. Tumor antigens are well known in the art and include, for example, a glioma-associated antigen, carcinoembryonic antigen (CEA), EGFRvIII, IL-llRa, IL-13Ra, EGFR, FAP, B7H3, Kit, CA LX, CS-1, MUC1, BCMA, bcr-abl, HER2, β-human chorionic gonadotropin, alphafetoprotein (AFP), ALK, CD19, TIM3, cyclin Bl, lectin-reactive AFP, Fos-related antigen 1, ADRB3, thyroglobulin, EphA2, RAGE-1, RUl, RU2, SSX2, AKAP-4, LCK, OY-TESl, PAX5, SART3, CLL-1, fucosyl GM1, GloboH, MN-CA IX, EPCAM, EVT6-AML, TGS5, human telomerase reverse transcriptase, plysialic acid, PLAC1, RUl, RU2 (AS), intestinal carboxyl esterase, lewisY, sLe, LY6K, HSP70, HSP27, mut hsp70-2, M-CSF, MYCN, RhoC, TRP-2, CYPIBI, BORIS, prostase, prostate-specific antigen (PSA), PAX3, PAP, NY-ESO-1, LAGE-la, LMP2, NCAM, p53, p53 mutant, Ras mutant, gplOO, prostein, OR51E2, PANX3, PSMA, PSCA, Her2/neu, hTERT, HMWMAA, HAVCR1, VEGFR2, PDGFR-beta, survivin and telomerase, legumain, HPV E6,E7, sperm protein 17, SSEA-4, tyrosinase, TARP, WT1, prostate-carcinoma tumor antigen-1 (PCTA-1), ML-IAP, MAGE, MAGE-A1, MAGE-A2, MAGE-C1, MAGE-C2, Annexin-A2, MAD-CT-1, MAD-CT-2, MelanA/MART 1, XAGE1, ELF2M, ERG (TMPRSS2 ETS fusion gene), NA17, neutrophil elastase, sarcoma translocation breakpoints, NY-BR-1, ephnnB2, CD20, CD22, CD24, CD30, TIM3, CD38, CD44v6, CD97, CD171, CD179a, androgen receptor, FAP, insulin growth factor (IGF)-I, IGFII, IGF-I receptor, GD2, o-acetyl-GD2, GD3, GM3, GPRC5D, GPR20, CXORF61, folate receptor (FRa), folate receptor beta, ROR1, Flt3, TAG72, TN Ag, Tie 2, TEM1, TEM7R, CLDN6, TSHR, UPK2, and mesothelin. In certain preferred embodiments, the tumor antigen is selected from folate receptor (FRa), mesothelin, EGFRvIII, IL-13Ra, CD123, CD19, TIM3, BCMA, GD2, CLL-1, CA-IX, MUCl, HER2, and any combination thereof.

Further non-limiting examples of tumor antigens include the following: Differentiation antigens such as tyrosinase, TRP-1, TRP-2 and tumor-specific multilineage antigens such as MAGE-1, MAGE-3, BAGE, GAGE-1, GAGE-2, pi 5; overexpressed embryonic antigens such as CEA; overexpressed oncogenes and mutated tumor-suppressor genes such as p53, Ras, HER-2/neu; unique tumor antigens resulting from chromosomal translocations; such as BCR-ABL, E2A-PRL, H4-RET, IGH-IGK, MYL-RAR; and viral antigens, such as the Epstein Barr virus antigens EBVA and the human papillomavirus (HPV) antigens E6 and E7. Other large, protein-based antigens include SCCA, GP73, FC-GP73, TSP-180, MAGE-4, MAGE-5, MAGE-6, RAGE, NY-ESO, pl85erbB2, pl80erbB-3, c-met, nm- 23H1, PSA, CA 19-9, CA 72-4, CAM 17.1, NuMa, K-ras, beta-Catenin, CDK4, Mum-1, p 15, p 16, 43-9F, 5T4, 791Tgp72, alpha-fetoprotein, beta-HCG, BCA225, BTAA, CA 125, CA 15-3\CA 27.29\BCAA, CA 195, CA 242, CA-50, CAM43, CD68\P1, CO-029, FGF-5, G250, Ga733\EpCAM, HTgp-175, M344, MA-50, MG7-Ag, MOV18, NB/70K, NY-CO-1, RCASl, SDCCAG1 6, TA-90\Mac-2 binding protein\cyclophilm C-associated protein, TAAL6, TAG72, TLP, TPS, GPC3, MUC16, TAG-72, LMP1, EBMA-1, BARF-1, CS1, CD319, HER1, B7H6, L1CAM, IL6, and MET.

The transmembrane domain (TD) connects the ectodomain (i.e., the extracellular domain) to the endodomain (i.e., the intracellular domain) and resides within the cell membrane when expressed by a cell. The transmembrane domain may be derived either from a natural or from a synthetic source. Where the source is natural, the domain may be derived from any membrane-bound or transmembrane protein. For example, the transmembrane region may be derived from (i.e., comprise at least the transmembrane region(s) of) the alpha, beta or zeta chain of the T-cell receptor, CD28, CD3 epsilon, CD45, CD4, CD5, CD8 (e.g., CD8 alpha, CD8 beta), CD9, CD16, CD22, CD33, CD37, CD64, CD80, CD86, CD134, CD137, or CD154, KIRDS2, OX40, CD2, CD27, LFA-1 (CD11a, CD18), ICOS (CD278), 4-1BB (CD137), GITR, CD40, BAFFR, HVEM (LIGHTR), SLAMF7, NKp80 (KLRF1), CD160, CD19, IL2R beta, IL2R gamma, IL7R α, ITGA1, VLA1, CD49a, ITGA4, IA4, CD49D, ITGA6, VLA-6, CD49f, ITGAD, CD11d, ITGAE, CD103, ITGAL, CD11a, LFA-1, ITGAM, CD11b, ITGAX, CD11c, ITGB1, CD29, ITGB2, CD18, LFA-1, ITGB7, TNFR2, DNAM1 (CD226), SLAMF4 (CD244, 2B4), CD84, CD96 (Tactile), CEACAM1, CRTAM, Ly9 (CD229), CD160 (BY55), PSGL1, CD100 (SEMA4D), SLAMF6 (NTB-A, Ly108), SLAM (SLAMF1, CD150, IPO-3), BLAME (SLAMF8), SELPLG (CD162), LTBR, and PAG/Cbp. Alternatively the transmembrane domain may be synthetic, in which case it will comprise predominantly hydrophobic residues such as leucine and valine. In some embodiments, a triplet of phenylalanine, tryptophan and valine will be found at each end of a synthetic transmembrane domain. A short oligo- or polypeptide linker, such as between 2 and 10 amino acids in length, may form the linkage between the transmembrane domain and the endoplasmic domain of the CAR.

The endodomain transmits an activation signal to the immune effector cell after antigen recognition, activating at least one of the normal effector functions of said immune effector cell. In certain embodiments, the effector function of a T cell, for example, may be cytolytic activity or helper activity, including the secretion of cytokines. Generally, the endodomain may contain an intracellular signaling domain (ISD) and, optionally, a co-stimulatory signaling region (CSR). The endodomain may comprise the ISD of a T cell receptor (TCR) and optional co-receptors. While the entire intracellular signaling domain can be employed, it is not necessary to use the entire chain. To the extent that a truncated portion of the intracellular signaling domain is used, such truncated portion may be used in place of the intact chain as long as it transduces the effector function signal. A "signaling domain (SD)", such as an ISD, generally contains cytoplasmic signaling sequences that regulate primary activation of the TCR complex that act in a stimulatory manner and are comprised of signaling motifs which are known as immunoreceptor tyrosine-based activation motifs (ITAMs). A signaling cascade is activated when the ITAM is phosphorylated. The term "co-stimulatory signaling region (CSR)" refers to intracellular signaling domains from costimulatory protein receptors, such as CD28, 41BB, and ICOS, that are able to enhance T-cell activation by T-cell receptors. In some embodiments, the endodomain contains an SD or a CSR, but not both. In these embodiments, an immune effector cell containing the disclosed CAR is only activated if another CAR (or a T-cell receptor) containing the missing domain also binds its respective antigen. Examples of ITAM-containing cytoplasmic signaling sequences include those derived from CD8, CD3ζ, CD3δ, CD3γ, CD3ε, CD32 (Fc gamma RIIa), DAP10, DAP12, CD79a, CD79b, FcγRIγ, FcyRIIIy, FcεRIβ (FCERIB), and FceRIy (FCERIG).

In particular embodiments, the intracellular signaling domain is derived from CD3 zeta (CD3ζ) (TCR zeta, GenBank accno. BAG36664.1). T-cell surface glycoprotein CD3 zeta (CD3ζ) chain, also known as T-cell receptor T3 zeta chain or CD247 (Cluster of Differentiation 247), is a protein that in humans is encoded by the *CD247* gene. The intracellular tails of the CD3 molecules contain a single ITAM, which is essential for the signaling capacity of the TCR. The intracellular tail of the ζ chain (CD3ζ) contains 3 ITAMs. In some embodiments, the ζ chain is a mutant ζ chain. For example, the mutant ζ chain comprises a mutation, such as a point mutation, in at least one ITAM so as to render said ITAM non-functional. In some such embodiments, either the membrane-proximal ITAM (ITAM1), the membrane-distal ITAM (C-terminal third ITAM, ITAM3), or both are non-functional. In further embodiments, either two membrane-proximal ITAMS (ITAM1 and ITAM2) or two membrane-distal ITAMS (ITAM2 and ITAM3) are non-functional. In yet further embodiments, only ITAM2 is non-functional. In some embodiments, the mutant ζ chain comprises a deletion (e.g., truncation) mutation such that at least one ITAM is missing. In some such embodiments, the ζ chain is missing the membrane-proximal ITAM (ITAM1), the membrane-distal ITAM (ITAM3), or both. In other embodiments, the ζ chain is missing either two membrane-proximal ITAMS (ITAM1 and ITAM2) or two membrane-distal ITAMS (ITAM2 and ITAM3). In further embodiments, the ζ chain is missing ITAM2. Methods to produce mutant CD3ζ are known to those skilled in the art (Bridgeman JS, et al., Clin Exp Immunol. 2014 Feb;175(2):258-67 and WO 2019/133969). Removing at least one ITAM from the introduced CAR may reduce CD3ζ-mediated apoptosis. Alternatively, removing at least one ITAM from the introduced CAR can reduce its size without loss of function.

In some embodiments, the CAR comprises a hinge sequence. A hinge sequence is a short sequence of amino acids that facilitates antibody flexibility (see, e.g., Woof et al., Nat. Rev. Immunol., 4(2): 89-99 (2004)). The hinge sequence may be positioned between the antigen recognition moiety (e.g., anti-CD19, -CD20, -CD22, or -CLEC4 scFv) and the transmembrane domain. The hinge sequence can be any suitable sequence derived or obtained from any suitable molecule. In some embodiments, for example, the hinge sequence is derived from a CD8a molecule or a CD28 molecule.

In preferred embodiments, the disclosed CAR is defined by the formula:

SP-TD-HG-TM-CSR-SD;

or

SP-TD-HG-TM-SD-C SR;

wherein "SP" represents an optional signal peptide,
wherein "TD" represents a targeting domain,
wherein "HG" represents an optional hinge domain (spacer domain),
wherein "TM" represents a transmembrane domain,
wherein "CSR" represents one or more co-stimulatory signaling regions,
wherein "SD" represents a signaling domain, and
wherein "-" represents a peptide bond or linker.

In some embodiments, the CAR contains one signaling domain. In other embodiments, the CAR contains one or more signaling domains (including co-stimulatory signaling domain). The one or more signaling domain may be a polypeptide selected from: CD8, CD3ζ, CD3δ, CD3γ, CD3e, FcyRI-y, FcγRIII-γ, FcεRIβ, FcεRIγ, DAP10, DAP12, CD32, CD79a, CD79b, CD28, CD3C, CD4, b2c, CD137 (41BB), ICOS, CD27, CD28δ, CD80, NKp30, OX40, and mutants thereof. For example, the endodomain of the CAR can be designed to comprise the CD3ζ signaling domain by itself or combined with any other desired cytoplasmic domain(s) useful in the context of the CAR of the invention. Alternatively, the cytoplasmic domain of the CAR can comprise a CD3ζ chain portion and a costimulatory signaling region. The costimulatory signaling region refers to a portion of the CAR comprising the intracellular domain of a costimulatory molecule. A costimulatory molecule is a cell surface molecule other than an antigen receptor or their ligands that is required for an efficient response of lymphocytes to an antigen. Examples of such molecules include CD27, CD28, 4-1BB (CD137), OX40, CD30, CD40, ICOS, lymphocyte function-associated antigen-1 (LFA-1), CD2, CD7, LIGHT, NKG2C, B7-H3, and a ligand that specifically binds with CD83, CD8, CD4, b2c, CD80, CD86, DAP10, DAP12, MyD88, BTNL3, NKG2D, and mutants thereof. Thus, while the CAR is exemplified primarily with CD28 as the co-stimulatory signaling element, other costimulatory elements, and mutants thereof, can be used alone or in combination with other co-stimulatory signaling elements. For example, in some such embodiments, the preferred CAR co-stimulatory signaling domain is the CD28 mutant domain known as "Mut06" as described in WO2019/010383.

In some embodiments, the CAR has more than one transmembrane domain, which can be a repeat of the same transmembrane domain, or can be different transmembrane domains.

In some embodiments, the CAR is a multi-chain CAR, as described in WO 2015/039523. A multi-chain CAR can comprise separate extracellular ligand binding and signaling domains in different transmembrane polypeptides. The signaling domains can be designed to assemble in juxtamembrane position, which forms flexible architecture closer to natural receptors, that confers optimal signal transduction. For example, the multi-chain CAR can comprise a part of an FCERI alpha chain and a part of an FCERI beta chain such that the FCERI chains spontaneously dimerize together to form a CAR.

Additional CAR constructs are described, for example, in Fresnak AD, et al. Engineered T cells: the promise and challenges of cancer immunotherapy. Nat Rev Cancer. 2016 Aug 23; 16(9) 566-581.

In certain embodiments, the CAR can be, for example (and without limitation), a TRUCK, a Universal CAR, a Self-driving CAR, an Armored CAR, a Self-destruct CAR, a Conditional CAR, a Marked CAR, a TenCAR, a Dual CAR, or a sCAR.

TRUCKs (T cells redirected for universal cytokine killing) co-express a chimeric antigen receptor (CAR) and an antitumor cytokine. Cytokine expression may be constitutive or induced by T cell activation. Targeted by CAR specificity, localized production of pro-inflammatory cytokines recruits endogenous immune cells to tumor sites and may potentiate an antitumor response.

Universal, allogeneic CAR T cells are engineered to no longer express endogenous T cell receptor (TCR) and/or major histocompatibility complex (MHC) molecules, thereby preventing graft-versus-host disease (GVHD) or rejection, respectively.

Self-driving CARs co-express a CAR and a chemokine receptor, which binds to a tumor ligand, thereby enhancing tumor homing.

In certain aspects disclosed herein, the invention employs immune checkpoint inhibition/blockade strategies. Immune checkpoint therapies target key regulators of the immune system that either stimulate or inhibit the immune response. Such immune checkpoints can be exploited in the disease state (e.g., by tumors) to evade attacks by the immune system. Checkpoint inhibitor studies have noted the activity of PD-1 inhibitor therapy (El-Khoueiry, Sangro et al., 2017) and the FDA has approved Nivolumab for second line treatment of HCC with an objective response rate of 20%. CAR T cells engineered to be resistant to immunosuppression (Armored CARs) may be genetically modified to no longer express various immune checkpoint molecules (e.g., cytotoxic T lymphocyte-associated antigen 4 (CTLA4) or programmed cell death protein 1 (PD-1)). Exemplary "Knockdown" and "Knockout" techniques include, but are not limited to, RNA interference (RNAi) (e.g., asRNA, miRNA, shRNA, siRNA, etc.) and CRISPR interference (CRISPRi) (e.g., CRISPR-Cas9). In certain embodiments, CAR T cells are engineered to express a dominant-negative form of a checkpoint molecule. In some such embodiments, the extracellular ligand-binding domain (i.e., ectodomain) of the immune checkpoint molecule is fused to a transmembrane membrane in order to compete for ligand binding. For example, the extracellular ligand-binding domain of PD-1 may be fused to a CD8 transmembrane domain, thus competing for PD-1 ligand from the target cell. In some embodiments, CAR T cells are engineered to express an immune checkpoint switch receptor to exploit the inhibitory immune checkpoint ligand present on a target cell. In such embodiments, the extracellular ligand-binding domain of the immune checkpoint molecule is fused to a signaling, stimulatory, and/or co-stimulatory domain. For example, the extracellular ligand-binding domain of PD-1 may be fused to a CD28 domain, thus providing CD28 costimulation while blocking PD-1 signaling. In further embodiments, the CAR T cells may be administered with an aptamer or a monoclonal antibody that blocks immune checkpoint signaling. In some such embodiments, the CAR T cells (e.g., CAR T cell therapy) are combined with a PD-1 blockade method, such as administration with PD-1/PD-L1 antagonistic aptamers or anti-PD-1/PD-L1 antibodies. In preferred embodiments, the CAR T cells and PD-1 pathway-blocking antibodies are administered conjointly. In further embodiments, the CAR T cells are engineered to express or express and secrete an immune checkpoint-blocking antibody, such as anti-PD-1 or anti-PD-L1, or fragments thereof. In yet further embodiments, the CAR T cells are administered with a vector (e.g., an engineered virus) that expresses an immune checkpoint-blocking molecule described herein.

A self-destruct CAR may be designed using RNA delivered by electroporation to encode the CAR. Alternatively, inducible apoptosis of the T cell may be achieved based on ganciclovir binding to thymidine kinase in gene-modified lymphocytes or the more recently described system of activation of human caspase 9 by a small-molecule dimerizer.

A conditional CAR T cell is by default unresponsive, or switched 'off', until the addition of a small molecule to complete the "circuit" (e.g., molecular pathway), enabling full transduction of both signal 1 and signal 2, thereby activating the CAR T cell. Alternatively, T cells may be engineered to express an adaptor-specific receptor with affinity for subsequently administered secondary antibodies directed at target antigen.

Marked CAR T cells express a CAR plus a tumor epitope to which an existing monoclonal antibody agent binds. In the setting of intolerable adverse effects, administration of the monoclonal antibody clears the CAR T cells and alleviates symptoms with no additional off-tumor effects.

A tandem CAR (TanCAR) T cell expresses a single CAR consisting of two linked single-chain variable fragments (scFvs) that have different affinities fused to intracellular co-stimulatory domain(s) and a CD3ζ domain. TanCAR T cell activation is achieved only when target cells co-express both targets.

A dual CAR T cell expresses two separate CARs with different ligand binding targets. By way of non-limiting example, one CAR may include only the CD3ζ domain while the other CAR includes only the co-stimulatory domain(s). In some such embodiments, the dual CAR T cell is activated when both targets are expressed on the tumor.

A safety CAR (sCAR) consists of an extracellular scFv fused to an intracellular inhibitory domain. sCAR T cells co-expressing a standard CAR become activated only when encountering target cells that possess the standard CAR target but lack the sCAR target.

Also disclosed are polynucleotides and polynucleotide vectors encoding the disclosed target-specific CARs that allow expression of said CARs in the disclosed immune effector cells (e.g., T cells).

Nucleic acid sequences encoding the disclosed CARs, and regions thereof, can be obtained using recombinant methods known in the art, such as, for example by screening libraries from cells expressing the gene, by deriving the gene from a vector known to include the same, or by isolating directly from cells and tissues containing the same, using standard techniques. Alternatively, the gene of interest can be produced synthetically, rather than cloned.

Expression of nucleic acids encoding CARs is typically achieved by operably linking a nucleic acid encoding the CAR polypeptide to a promoter, and incorporating the construct into an expression vector. Typical cloning vectors contain transcription and translation terminators, initiation sequences, and promoters useful for regulation of the expression of the desired nucleic acid sequence.

The disclosed nucleic acid can be cloned into a number of types of vectors. For example, the nucleic acid can be cloned into a vector including, but not limited to a plasmid, a phagemid, a phage derivative, an animal virus, and a cosmid. Vectors of particular interest include expression vectors, replication vectors, probe generation vectors, and sequencing vectors.

Further, the expression vector may be provided to a cell in the form of a viral vector. Viral vector technology is well known in the art and is described, for example, in Sambrook et al. (2001, Molecular Cloning: A Laboratory Manual, Cold Spring Harbor Laboratory, New York), and in other virology and molecular biology manuals. Viruses, which are useful as vectors include, but are not limited to, retroviruses (e.g., gammaretrovirus), adenoviruses, adeno-associated viruses, herpes viruses, and lentiviruses. In general, a suitable vector contains an origin of replication functional in at least one organism, a promoter sequence, convenient restriction endonuclease sites, and one or more selectable markers. In some embodimens, the polynucleotide vectors are lentiviral or retroviral vectors. Preferably, the polynucleotide vectors are gammaretroviral vectors.

A number of viral based systems have been developed for gene transfer into mammalian cells. For example, retroviruses provide a convenient platform for gene delivery systems. A selected gene can be inserted into a vector and packaged in retroviral particles using techniques known in the art. The recombinant virus can then be isolated and delivered to cells of the subject either in vivo or ex vivo.

One example of a suitable promoter is the immediate early cytomegalovirus (CMV) promoter sequence. This promoter sequence is a strong constitutive promoter sequence capable of driving high levels of expression of any polynucleotide sequence operatively linked thereto. Another example of a suitable promoter is Elongation Growth Factor-1α (EF-1α). However, other constitutive promoter sequences may also be used, including, but not limited to the simian virus 40 (SV40) early promoter, MND (myeloproliferative sarcoma virus) promoter, mouse mammary tumor virus (MMTV), human immunodeficiency virus (HIV) long terminal repeat (LTR) promoter, MoMuLV promoter, an avian leukemia virus promoter, an Epstein-Barr virus immediate early promoter, a Rous sarcoma virus promoter, as well as human gene promoters such as, but not limited to, the actin promoter, the myosin promoter, the hemoglobin promoter, and the creatine kinase promoter. The promoter can alternatively be an inducible promoter. Examples of inducible promoters include, but are not limited to a metallothionine promoter, a glucocorticoid promoter, a progesterone promoter, and a tetracycline promoter.

Additional promoter elements, e.g*.,* enhancers, regulate the frequency of transcriptional initiation. Typically, these are located in the region 30-110 bp upstream of the start site, although a number of promoters have recently been shown to contain functional elements downstream of the start site as well. The spacing between promoter elements frequently is flexible, so that promoter function is preserved when elements are inverted or moved relative to one another.

Reporter genes are used for identifying potentially transfected cells and for evaluating the functionality of regulatory sequences. In general, a reporter gene is a gene that is not present in or expressed by the recipient organism or tissue and that encodes a polypeptide whose expression is manifested by some easily detectable property, e.g., enzymatic activity. Expression of the reporter gene is assayed at a suitable time after the DNA has been introduced into the recipient cells. Suitable reporter genes may include genes encoding luciferase, beta-galactosidase, chloramphenicol acetyl transferase, secreted alkaline phosphatase, or the green fluorescent protein gene. Suitable expression systems are well known and may be prepared using known techniques or obtained commercially. In general, the construct with the minimal 5' flanking region showing the highest level of expression of reporter gene is identified as the promoter. Such promoter regions may be linked to a reporter gene and used to evaluate agents for the ability to modulate promoter-driven transcription.

Methods of introducing and expressing genes into a cell are known in the art. In the context of an expression vector, the vector can be readily introduced into a host cell, e.g*.,* mammalian, bacterial, yeast, or insect cell by any method in the art. For example, the expression vector can be transferred into a host cell by physical, chemical, or biological means.

Physical methods for introducing a polynucleotide into a host cell include calcium phosphate precipitation, lipofection, particle bombardment, microinjection, electroporation, and the like. Methods for producing cells comprising vectors and/or exogenous nucleic acids are well-known in the art. See, for example, Sambrook et al. (2001, Molecular Cloning: A Laboratory Manual, Cold Spring Harbor Laboratory, New York).

Biological methods for introducing a polynucleotide of interest into a host cell include the use of DNA and RNA vectors. Viral vectors, and especially retroviral vectors, have become the most widely used method for inserting genes into mammalian, e.g., human cells.

Chemical means for introducing a polynucleotide into a host cell include colloidal dispersion systems, such as macromolecule complexes, nanocapsules, microspheres, beads, and lipid-based systems including oil-in-water emulsions, micelles, mixed micelles, and liposomes. An exemplary colloidal system for use as a delivery vehicle in vitro and in vivo is a liposome (e.g., an artificial membrane vesicle).

In the case where a non-viral delivery system is utilized, an exemplary delivery vehicle is a liposome. In another aspect, the nucleic acid may be associated with a lipid. The nucleic acid associated with a lipid may be encapsulated in the aqueous interior of a liposome, interspersed within the lipid bilayer of a liposome, attached to a liposome via a linking molecule that is associated with both the liposome and the oligonucleotide, entrapped in a liposome, complexed with a liposome, dispersed in a solution containing a lipid, mixed with a lipid, combined with a lipid, contained as a suspension in a lipid, contained or complexed with a micelle, or otherwise associated with a lipid. Lipid, lipid/DNA or lipid/expression vector associated compositions are not limited to any particular structure in solution. For example, they may be present in a bilayer structure, as micelles, or with a "collapsed" structure. They may also simply be interspersed in a solution, possibly forming aggregates that are not uniform in size or shape. Lipids are fatty substances which may be naturally occurring or synthetic lipids. For example, lipids include the fatty droplets that naturally occur in the cytoplasm as well as the class of compounds which contain long-chain aliphatic hydrocarbons and their derivatives, such as fatty acids, alcohols, amines, amino alcohols, and aldehydes. Lipids suitable for use can be obtained from commercial sources. For example, dimyristyl phosphatidylcholine ("DMPC") can be obtained from Sigma, St. Louis, Mo.; dicetyl phosphate ("DCP") can be obtained from K & K Laboratories (Plainview, N.Y.); cholesterol ("Choi") can be obtained from Calbiochem-Behring; dimyristyl phosphatidylglycerol ("DMPG") and other lipids may be obtained from Avanti Polar Lipids, Inc, (Birmingham, Ala.).

### Selection

In order to assess the expression of a CAR polypeptide or portions thereof, the expression vector to be introduced into a cell (e.g., an antigen-specific T cell) can also contain either a selectable marker gene or a reporter gene or both to facilitate identification and selection of expressing cells from the population of cells sought to be transfected or infected through viral vectors. In other embodiments, the selectable marker may be carried on a separate piece of DNA and used in a co-transfection procedure. Both selectable markers and reporter genes may be flanked with appropriate regulatory sequences to enable expression in the host cells. In some embodiments, the CAR-encoding vector comprises a lentiviral vector. In some preferred embodiments the CAR-encoding vector comprises a gammaretroviral vector. Most preferably, the CAR-encoding vector comprises a nucleic acid sequence encoding a selectable marker or physical tag that allows selection of only those cells expressing the CAR(s) encoded by said vector. Useful selectable markers include, but are not limited to, antibiotic-resistance genes, reporter genes, or expression of a physical tag. Physical tags may include, but are not limited to, truncated cell surface peptides (e.g., a cell surface receptor that lacks intracellular signaling domains) and can be selected with an appropriate antibody, using methods known in the art. In some embodiments, the CAR-encoding vector comprises a nucleic acid sequence encoding an antibiotic-resistance gene such as bor blasticidin resistance. In some such embodiments, the CAR T cells (e.g., the antigen-specific CAR T cells) are cultured in the presence of the selectable marker (e.g., blasticidin), thereby allowing for selection and expansion of CAR-expressing T cells described herein.

### Therapeutic Methods

In some embodiments, the methods provided herein are directed to treating a cancer and/or an autoimmune disorder in a subject by administering to the subject autologous or allogeneic CAR-T cells as provided herein.

The methods of the present disclosure rely, in part, on the principles of T cell restriction to specific target antigens. For example, and without limitation, products and preparations comprising donor-derived CTLs targeted against antigens expressed by a virus, such as Epstein-Barr Virus (EBV), can be elicited on targeted antigen-presenting (e.g., stimulator) cell lines, including EBV-transformed B Lymphoblasts (BLCLs). With respect to the disclosed virus-antigen-specific CAR T cell preparations (e.g., anti-CD19-CD28-CAR-EBV-CTLs), the target cells and/or recipients of the CAR-T products made by the methods provided herein, may be autologous or allogeneic. In the case of allogeneic targets, the human leukocyte antigen (HLA) identity of the donor must be known and matched to the HLA identity of the target (i.e., cultured cell lines and/or recipients).

A preparation of CAR T cells may have a mixture of antigen-specific and non-specific CTLs. Thus, to quantify alloreactive CTLs (e.g., antigen-specific CTLs with or without a CAR), a reaction against HLA-mismatched targets is elicited. Ideally, cell lysis should be minimal, or below a predetermined threshold. Further, the level of expandable CTLs or CAR-expressing CTLs may be quantified by limiting dilution assay and dilution of cytotoxicity against allogeneic targets.

Target cells for the assays disclosed herein are typically selected for their uniform phenotype and availability in large quantities. In certain embodiments, the target cell lines are antigen-presenting cells (APCs). In some such embodiments, the target cell lines are B cells, antigen-presenting T-cells, dendritic cells, or artificial antigen-presenting cells (e.g., aK562 cells). In certain embodiments, the target cell lines comprise peripheral blood mononuclear cells (PBMCs). In some such embodiments, the target cell lines are lymphoblasts. In certain preferred embodiments, the target cell lines are virally transformed. In preferred embodiments, the target cell lines comprise each of Phytohemagglutinin-stimulated peripheral blood lymphocytes (PHA-blasts/ PHAbs) and Epstein-Barr virus-transformed B-lymphoblastoid cell lines (BLCLs). As exemplified herein for the assessment of CAR-expressing antigen-specific CTLs, BLCLs have the advantage of being larger cells with high viability and the ability to retain significant lactate dehydrogenase (LDH) and/or large intracellular chromium (Cr) reservoirs, making them favorable for cytotoxicity assessment via Cr or LDH release assays. However, in the case of EBV antigen, BLCLs (such as those used as APCs) are EBV antigen-positive, which allows EBV antigen-specific cross-presentation to T-cell receptors (TCRs) specific to unmatched HLA alleles. PHA-blasts have the advantage of being EBV-antigen-negative and cannot cross-present EBV antigens to TCRs. Still, PHA-blasts are smaller and generally more fragile cells. This results in smaller reservoirs and permeability of reporter that ultimately provide higher probability of false-positive cytotoxicity results. Thus, BLCLs and PHA-blasts may be used as target pairs, derived from the same donor. In some such embodiments, the target cells are derived from the potential recipient of an allogeneic CAR T cell preparation described herein, so as to guide appropriate selection of a CAR T cell preparation to be administered.

In certain embodiments, if the preparation induces lysis in a plurality of target cell lines above a predetermined threshold, the method further comprises quantifying in the preparation the frequency of expandable CAR-T CTLs capable of lysing a target cell line, wherein the sample is identified as not being clinically alloreactive if it contains a level of the expandable CAR-T CTLs that is at or below a predetermined threshold. In certain embodiments, prior to quantifying the frequency of cytolytic, expandable CAR-T CTLs, the preparation induces more than 15% lysis in each of the allogeneic PHA-blast cell line and the allogeneic cell line.

In some embodiments, quantifying the frequency of expandable CAR-T CTLs in the preparation comprises evaluating cytolytic function after a period of CAR-T CTL expansion, e.g., by performing limiting dilution analysis. In further embodiments, evaluating cytolytic function comprises performing a detection method such as radioactive chromium (e.g., ⁵¹Cr) release or lactate dehydrogenase (LDH) release.

In some embodiments, target cell lines comprise allogeneic cells. In some such embodiments, target cells carry Human Leukocyte Antigen (HLA) alleles that do not match the HLA alleles to which the CAR-T CTLs of the preparation are restricted. In other such embodiments, the target cells carry HLA alleles that are a partial match to the HLA alleles to which the CAR-T CTLs of the preparation are restricted. In still other such embodiments, the target cells carry HLA alleles that are a match to the HLA alleles to which the CAR-T CTLs of the preparation are restricted. In preferred embodiments, the CAR-T CTLs are restricted to HLA alleles of the target cells which encode MHC Class I proteins.

In other embodiments, the target cell lines comprise autologous cells. In some such embodiments the CAR-T CTL preparation is identified as suitable for use against target cells by confirming the ability of the preparation to lyse two or more autologous target cell lines at, above, or below predetermined thresholds.

In certain embodiments, the autologous or allogeneic CAR-T cells comprise central memory T cells (T_{CM} cells), *e.g.,* at least 60%, 70%, 80% of the cells are Tcm cells. In some embodiments, the autologous or allogeneic CAR-T cells comprise a ratio of central memory T cells to effector memory cells (T_{CM}:T_{EM}) from at least 1:1 to at least 3:1, *e.g.* at least 1:1, 1.4:1, 2.5:1, or 3:1). In some such embodiments, the autologous or allogeneic CAR-T cells are predominantly CD4⁺ T cells. In some embodiments, the autologous or allogeneic CAR-T cells comprise at least 80% CD4⁺ CAR-T cells and at least 15% CD8⁺ CAR-T cells). In some embodiments, the autologous or allogeneic CAR-T cells comprise a ratio of CD4⁺ T cells to CD8⁺ T cells from at least 1:1 to at least 3:1, *e.g.,* at least 1:1, 1.4:1, 2.5:1, or 3:1. In some of the methods described herein, allogenic T cells are selected from a cell bank *(e.g.,* a pre-generated third party donor derived bank of epitope-specific T cells).

In some embodiments, the methods provided herein can be used to treat any autoimmune disease. Examples of autoimmune diseases include, for example, glomerular nephritis, arthritis, dilated cardiomyopathy-like disease, ulcerous colitis, Sjogren syndrome, Crohn disease, systemic erythematodes, chronic rheumatoid arthritis, juvenile rheumatoid arthritis, Still's diease, multiple sclerosis, psoriasis, allergic contact dermatitis, polymyositis, pachyderma, periarteritis nodosa, rheumatic fever, vitiligo vulgaris, Behcet disease, Hashimoto disease, Addison disease, dermatomyositis, myasthenia gravis, Reiter syndrome, Graves' disease, anaemia perniciosa, sterility disease, pemphigus, autoimmune thrombopenic purpura, autoimmune hemolytic anemia, active chronic hepatitis, Addison's disease, anti-phospholipid syndrome, atopic allergy, autoimmune atrophic gastritis, achlorhydra autoimmune, celiac disease, Cushing's syndrome, dermatomyositis, discoid lupus erythematosus, Goodpasture's syndrome, Hashimoto's thyroiditis, idiopathic adrenal atrophy, idiopathic thrombocytopenia, insulin-dependent diabetes, Lambert-Eaton syndrome, lupoid hepatitis, lymphopenia, mixed connective tissue disease, pemphigoid, pemphigus vulgaris, pernicious anemia, phacogenic uveitis, polyarteritis nodosa, polyglandular autosyndromes, primary biliary cirrhosis, primary sclerosing cholangitis, Raynaud's syndrome, relapsing polychondritis, Schmidt's syndrome, limited scleroderma (or crest syndrome), sympathetic ophthalmia, systemic lupus erythematosis, Takayasu's arteritis, temporal arteritis, thyrotoxicosis, type b insulin resistance, type I diabetes, ulcerative colitis and Wegener's granulomatosis.

In some embodiments, the methods provided herein are used to treat MS. In some embodiments, the MS is relapsing-remitting MS, secondary progressive MS, primary progressive MS or progressively relapsing MS.

In some embodiments, the methods provided herein are used to treat a SAD. For example, in certain embodiments, the methods provided herein are used to treat rheumatoid arthritis, systemic lupus erythematosus and/or Sjögren's syndrome.

In some embodiments, the methods provided herein are used to treat IBD. For example, in certain embodiments the methods provided herein are used to treat Crohn's disease (regional bowel disease, *e.g.,* inactive and active forms), celiac disease *(e.g.,* inactive or active forms) and/or ulcerative colitis (*e.g*., inactive and active forms). In some embodiments, the methods provided herein are used to treat irritable bowel syndrome, microscopic colitis, lymphocytic-plasmocytic enteritis, coeliac disease, collagenous colitis, lymphocytic colitis, eosinophilic enterocolitis, indeterminate colitis, infectious colitis (viral, bacterial or protozoan, *e.g.* amoebic colitis) *(e.g.,* clostridium dificile colitis), pseudomembranous colitis (necrotizing colitis), ischemic inflammatory bowel disease, Behcet's disease, sarcoidosis, scleroderma, IBD-associated dysplasia, dysplasia associated masses or lesions, and/or primary sclerosing cholangitis.

In some embodiments, provided herein are methods of treating a cancer in a subject by administering to the subject a therapeutic CAR-T cell preparation as described herein.

In some embodiments, the methods provided herein can be used to treat any cancer. For example, in some embodiments, the methods and CAR-T cells described herein may be used to treat any cancerous or pre-cancerous tumor. In some embodiments, the cancer includes a solid tumor. In some embodiments, cancers that may be treated by methods and compositions provided herein include, but are not limited to, cancer cells from the bladder, blood, bone, bone marrow, brain, breast, colon, esophagus, gastrointestine, gum, head, kidney, liver, lung, nasopharynx, neck, ovary, prostate, skin, stomach, testis, tongue, or uterus. In addition, the cancer may specifically be of the following histological type, though it is not limited to these: neoplasm, malignant; carcinoma; carcinoma, undifferentiated; giant and spindle cell carcinoma; small cell carcinoma; papillary carcinoma; squamous cell carcinoma; lymphoepithelial carcinoma; basal cell carcinoma; pilomatrix carcinoma; transitional cell carcinoma; papillary transitional cell carcinoma; adenocarcinoma; gastrinoma, malignant; cholangiocarcinoma; hepatocellular carcinoma; combined hepatocellular carcinoma and cholangiocarcinoma; trabecular adenocarcinoma; adenoid cystic carcinoma; adenocarcinoma in adenomatous polyp; adenocarcinoma, familial polyposis coli; solid carcinoma; carcinoid tumor, malignant; branchiolo-alveolar adenocarcinoma; papillary adenocarcinoma; chromophobe carcinoma; acidophil carcinoma; oxyphilic adenocarcinoma; basophil carcinoma; clear cell adenocarcinoma; granular cell carcinoma; follicular adenocarcinoma; papillary and follicular adenocarcinoma; nonencapsulating sclerosing carcinoma; adrenal cortical carcinoma; endometrioid carcinoma; skin appendage carcinoma; apocrine adenocarcinoma; sebaceous adenocarcinoma; ceruminous adenocarcinoma; mucoepidermoid carcinoma; cystadenocarcinoma; papillary cystadenocarcinoma; papillary serous cystadenocarcinoma; mucinous cystadenocarcinoma; mucinous adenocarcinoma; signet ring cell carcinoma; infiltrating duct carcinoma; medullary carcinoma; lobular carcinoma; inflammatory carcinoma; mammary paget's disease; acinar cell carcinoma; adenosquamous carcinoma; adenocarcinoma w/squamous metaplasia; malignant thymoma; malignant ovarian stromal tumor; malignant thecoma; malignant granulosa cell tumor; and malignant roblastoma; sertoli cell carcinoma; malignant leydig cell tumor; malignant lipid cell tumor; malignant paraganglioma; malignant extra-mammary paraganglioma; pheochromocytoma; glomangiosarcoma; malignant melanoma; amelanotic melanoma; superficial spreading melanoma; malignant melanoma in giant pigmented nevus; epithelioid cell melanoma; malignant blue nevus; sarcoma; fibrosarcoma; malignant fibrous histiocytoma; myxosarcoma; liposarcoma; leiomyosarcoma; rhabdomyosarcoma; embryonal rhabdomyosarcoma; alveolar rhabdomyosarcoma; stromal sarcoma; malignant mixed tumor; mullerian mixed tumor; nephroblastoma; hepatoblastoma; carcinosarcoma; malignant mesenchymoma; malignant brenner tumor; malignant phyllodes tumor; synovial sarcoma; malignant mesothelioma; dysgerminoma; embryonal carcinoma; malignant teratoma; malignant struma ovarii; choriocarcinoma; malignant mesonephroma; hemangiosarcoma; malignant hemangioendothelioma; kaposi's sarcoma; malignant hemangiopericytoma; lymphangiosarcoma; osteosarcoma; juxtacortical osteosarcoma; chondrosarcoma; malignant chondroblastoma; mesenchymal chondrosarcoma; giant cell tumor of bone; ewing's sarcoma; malignant odontogenic tumor; ameloblastic odontosarcoma; malignant ameloblastoma; ameloblastic fibrosarcoma; malignant pinealoma; chordoma; malignant glioma; ependymoma; astrocytoma; protoplasmic astrocytoma; fibrillary astrocytoma; astroblastoma; glioblastoma; oligodendroglioma; oligodendroblastoma; primitive neuroectodermal; cerebellar sarcoma; ganglioneuroblastoma; neuroblastoma; retinoblastoma; olfactory neurogenic tumor; malignant meningioma; neurofibrosarcoma; malignant neurilemmoma; malignant granular cell tumor; malignant lymphoma; Hodgkin's disease; Hodgkin's lymphoma; paragranuloma; small lymphocytic malignant lymphoma; diffuse large cell malignant lymphoma; follicular malignant lymphoma; mycosis fungoides; other specified non-Hodgkin's lymphomas; malignant histiocytosis; multiple myeloma; mast cell sarcoma; immunoproliferative small intestinal disease; leukemia; lymphoid leukemia; plasma cell leukemia; erythroleukemia; lymphosarcoma cell leukemia; myeloid leukemia; basophilic leukemia; eosinophilic leukemia; monocytic leukemia; mast cell leukemia; megakaryoblastic leukemia; myeloid sarcoma; and hairy cell leukemia. In some such preferred embodiments, the compositions and methods provided herein can be used to treat B-cell malignancies (*e.g.*, CD19⁺ malignancies) including chronic lymphocytic leukemia (CLL), ALL, and many non Hodgkin lymphomas.

In some embodiments, the methods provided herein are used to treat EBV-associated cancer. In some embodiments, the EBV-associated cancer is EBV-associated NPC. In some embodiments, the EBV associated cancer is post-transplant lymphoproliferative disorder (PTLD), NK/T cell lymphoma, EBV⁺ gastric cancer, or EBV⁺ leiomyosarcoma.

In some embodiments, the subject has been exposed to a virus (e.g., EBV) such that virus particles are detectable in the subject's blood. In some embodiments, the method further comprises measuring viral load in the subject (*e.g*., before or after administering the peptide specific CAR-T cells to the subject). Determining viral load in a subject may be a good prognostic marker for immunotherapy effectiveness. In some embodiments, selecting CAR-T cells further comprises determining the number of viral DNA copies in the subject *(e.g.,* in a tissue or blood sample). In some embodiments, viral load is measured two or more times. Actual dosage levels of the active ingredients in the pharmaceutical compositions provided herein may be varied so as to obtain an amount of the active ingredient which is effective to achieve the desired therapeutic response for a particular patient, composition, and mode of administration, without being toxic to the patient.

The selected dosage level will depend upon a variety of factors including the activity of the particular agent employed, the route of administration, the time of administration, the rate of excretion or metabolism of the particular compound being employed, the duration of the treatment, other drugs, compounds and/or materials used in combination with the particular compound employed, the age, sex, weight, condition, general health and prior medical history of the patient being treated, and like factors well known in the medical arts.

In some embodiments, the methods described herein include selecting allogeneic T cells from a cell bank *(e.g.,* a pre-generated third party donor derived bank of epitope specific T cells). In some embodiments, the T cells are selected because they express a TCR restricted to a class I MHC that is encoded by an HLA allele that is present in the subject. In some embodiments, the T cells are selected if the T cells and subject share at least 2 *(e.g.,* at least 3, at least 4, at least 5, at least 6) HLA alleles and the T cells are restricted through a shared HLA allele. In some embodiments, the method comprises testing the TCR repertoire of the pre-generated third-party-donor-derived epitope-specific T cells (*i.e.,* allogeneic T cells) with flow cytometry. In some embodiments, epitope-specific T cells are detected using a tetramer assay, an ELISA assay, a western blot assay, a fluorescent microscopy assay, an Edman degradation assay and/or a mass spectrometry assay (*e.g.*, protein sequencing). In some embodiments, the TCR repertoire is analyzed using a nucleic acid probe, a nucleic acid amplification assay and/or a sequencing assay.

In some embodiments, provided herein are compositions (*e.g.*, therapeutic compositions) comprising T cells and/or APCs provided herein used to treat and/or prevent an autoimmune disease in a subject by administering to the subject an effective amount of the composition. In some aspects, provided herein are methods of treating autoimmune disorders using a composition (*e.g*., a pharmaceutical composition, such compositions comprising allogeneic CTLs). In some embodiments, the composition includes a combination of multiple (*e.g.*, two or more) CTLs provided herein.

### EXAMPLES

### Example 1: Comparison of CAR transduction of EBV CTLs derived from PBMCs and isolated T cells

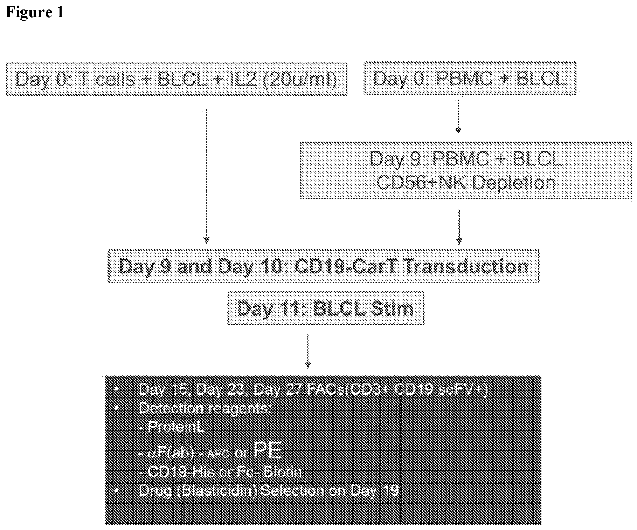

Frozen PBMC from healthy donors were thawed and recovered into RPMI medium. Cells were split into two fractions; 1/3 of cells (Stimulators) were infected with AdE1-LMPpoly adenovirus for one hour at 37°C. Stimulators were then washed twice and resuspended in RPMI/AB serum culture medium and irradiated at 2500 cGy (2500 rads). The remaining 2/3 of cells (Responders) were either transferred to RPMI/AB serum medium and held at 37°C until they could be mixed with the Stimulators (e.g., PBMC-derived BLCL) or were used to isolate CD3⁺ T cells that were then cultured with the Stimulator PBMC (see Figure 1).

### PBMC activation

On day 0, cultures were initiated in 6 well (10cm²) GRex culture plates at a of 9×10⁶ irradiated Stimulator PBMC cells to 2.1×10⁷ Responder PBMC cells and returned to 37°C, tissue culture incubation. Though optional, on days 9 and 10, cell cultures were depleted of CD56⁺, NK (Natural Killer) cells, prior to transduction.

### Activation of isolated CD3⁺T cells

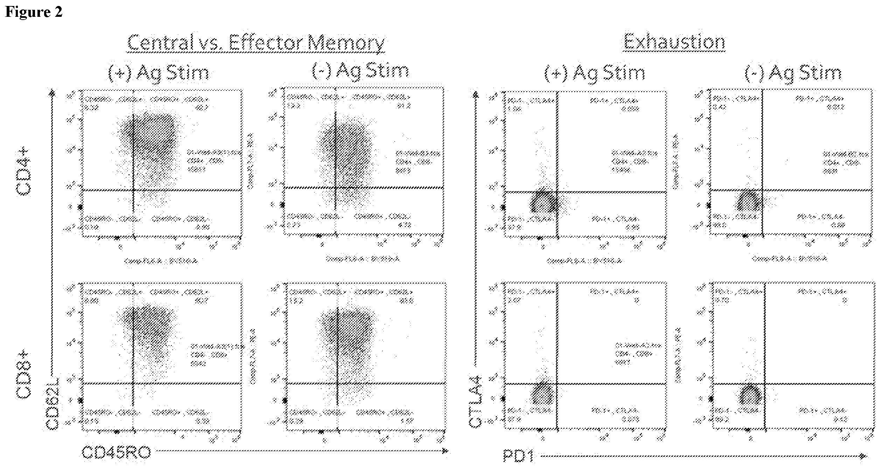

On day 0, T cells were isolated (i.e., enriched) from PBMC by means known in the art (e.g., live FACS or anti-CD3-coated magnetic beads). T cells were initiated in 6 well (10cm²) GRex culture plates containing media comprising 20U/ml IL2, at a ratio of 1 T cell / 4 Stimulator PBMC cells and returned to 37°C, tissue culture incubation. On days 9 and 10, cell cultures were transduced to express CARs (as above, NK cell depletion was optional prior to transduction). Sensitizing the starting material (e.g., following CD3⁺ enrichment) to viral antigens, such as EBV, results in an increase in the percentage of central memory phenotype T cells in the resultant population. (See Figure 2, left side) These central memory T cells advantageously persist over time while markers of T cell exhaustion (PD1 and CTLA4) remain low in virally-sensitized cells. (Figure 2 right side).

### CAR Transduction

On days 9 and 10 isolated T cell and PBMC cultures were transduced with recombinant virus encoding anti-CD19 CAR (and blasticidin selection marker). Briefly, retronectin (0.5 mL 10µg/mL) was added to each well of a 24-well non-tissue culture treated plate for each stimulation condition and incubated 2 hours at room temperature. The retronectin was then removed by aspiration, replaced with 0.5 mL blocking buffer to each well, and plates incubated 30 minutes at room temperature. Plates were then washed with wash buffer. Virus encoding anti-CD19 CAR were thawed at room temperature and added to plates. Plates were wrapped in paraffin film and centrifuged at 2000xg for 2 hours at 32°C. Viral supernatant was aspirated and 1 ml of prepared cell suspension from each stimulation group (0.5 × 106 cells/mL resuspended in YH5 media) was added. Plates were centrifuged at 1000xg for 15 minutes at 32°C and incubated at 37°C, 5% CO2 overnight. Cells for each stimulation condition were then removed and pooled for counting. Cells were resuspended in fresh YH5 media at 0.5-1.0×10⁶ cells/mL.

On day 11, each stimulation condition (i.e. of isolated T cells or of Responder PBMC) were returned to culture with irradiated Stimulator PBMC. Samples were taken at each of Days 15, 23 and 27 for Fluorescence-Activated Cell Sorting (FACS) *(e.g.,* for CD3⁺, scFV⁺ cells). Drug selection (blasticidin) was induced on day 19.

### Results

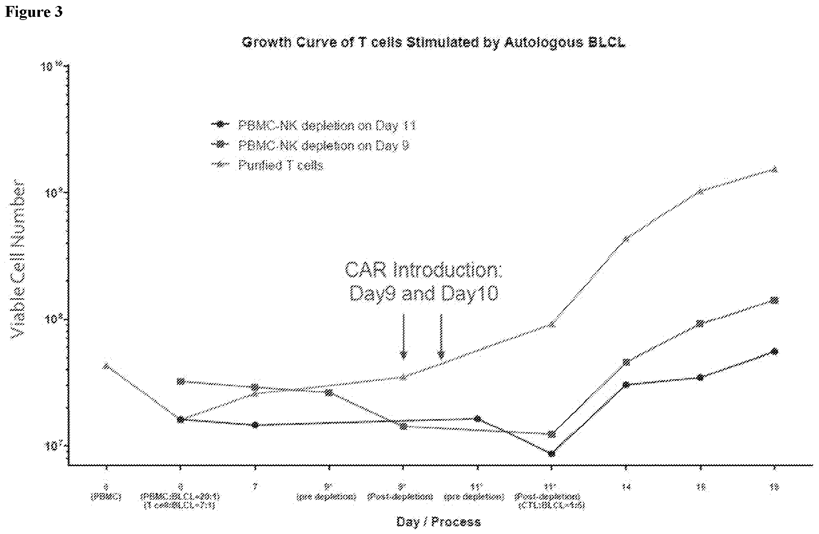

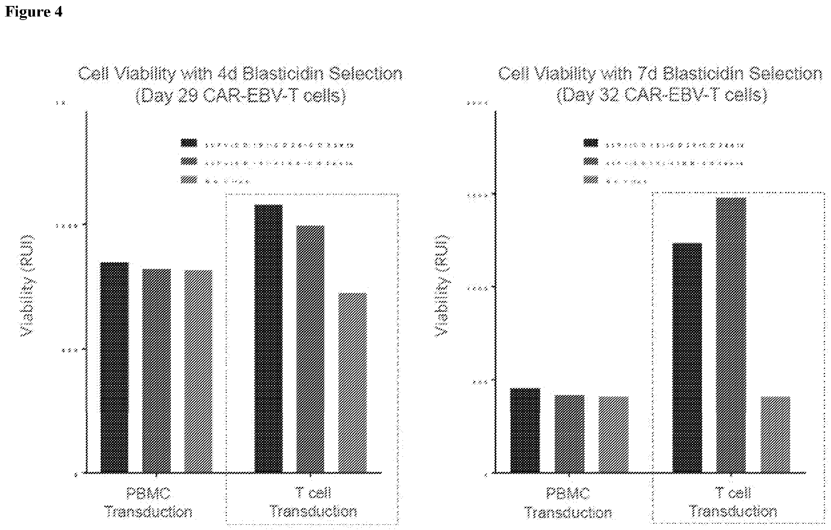

EBV-CTLs derived from isolated T cells demonstrate improved viability and proliferative capacity. CD3⁺ enriched starting material has a greater than 10-fold yield over conventional expansion and transduction conditions, demonstrating significantly improved viability and proliferative capacity. (See Figure 3) The efficiency of downstream CAR vector transduction is dependent upon proliferative capacity. Thus, higher viability and proliferative capacity in the antigen-stimulated T cells results in improved downstream CAR transduction efficiency. When compared to transduction in a crude PBMC culture, CAR⁺ EBV CTLs derived from an initial CD3⁺ enrichment step demonstrate a significant enhancement of downstream CAR transduction efficiency post BLCL stimulation and also show greater cell viability in response to blasticidin selection. (See Figure 4)

### Example 2: Evaluation of the memory T cell phenotype on anti-CD19-CAR-EBV-CTL following BLCL stimulation

Standard anti-CD3/CD28 bead-based stimulation is widely used in the field as a method to expand T cells in vitro prior to transduction with CAR vectors. The following experiment was undertaken to determine the impact of various CAR-T cell stimuli amenable to a high-yield manufacturing process on the memory T cell immune-phenotype for a final therapeutic product.

### Study design and procedure

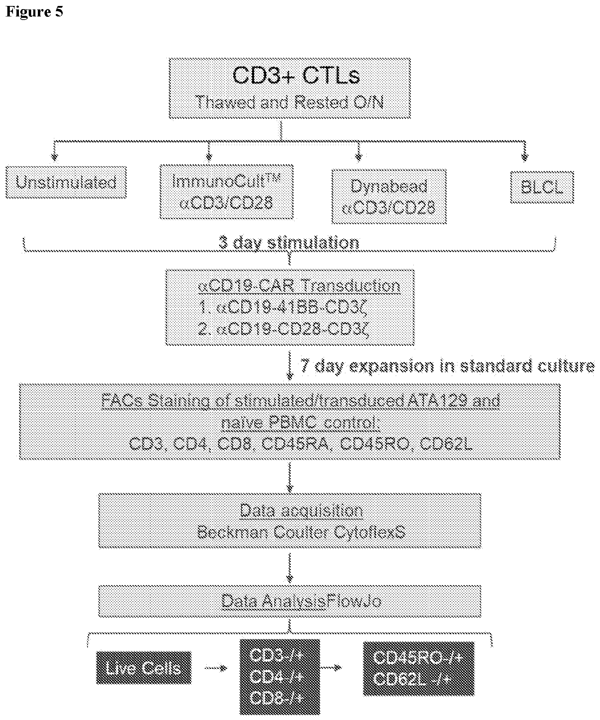

Isolated CD3⁺, EBV-antigen specific T cells were stimulated and expanded in 4 different culture conditions for 3 days;
1. Unstimulated,
2. soluble anti-CD3/CD28,
3. anti-CD3/CD28 magnetic beads, and
4. with BLCL cells.
Following 3 days of stimulation, each culture was transduced with a viral vector encoding a chimeric antigen receptor (CAR) and returned to incubation for 7 days in standard (YH media) culture to allow for expansion of the transduced T cells. Cells were then harvested and cell phenotypes analyzed by cell staining and fluorescence activated cell sorting (FACS) as outlined in Figure 5).

### Cell lines and culture conditions

CD3⁺, EBV-antigen specific T cells were removed from liquid nitrogen storage, thawed, and suspended in YH media at a concentration of 2 × 10⁶ cells/mL with 100 IU/mL IL-2. The suspension was plated in a 24-well plate with 2mLs of the suspension per well and incubated overnight. Following this overnight recovery, cells were counted and divided into the four different treatment groups as described in Figure 5 (see also Table 2) with 6 × 10⁶ cells per group, at a concentration of 1 × 10⁶ cells/mL.

**Table 2: Stimulation conditions to evaluate memory phenotype on T cells following stimulation and CAR transduction**

| **Number** | **Stimulation Condition** | **Lentivirus MOI 15** |
|---|---|---|
| **1** | Unstimulated | 28 |
| **2** | Unstimulated | 4-1BB |
| **3** | CD3/CD28 Dynabeads (1: 1, bead to cell) | 28 |
| **4** | CD3/CD28 Dynabeads (1:1, bead to cell) | 4-1BB |
| **5** | CD3/CD28 ImmunoCult (25 µl/mL) | 28 |
| 6 | CD3/CD28 ImmunoCult (25 µl/mL) | 4-1BB |
| 7 | BLCL | 28 |
| 8 | BLCL | 4-1BB |
| 9 | BLCL | None |

Stimulation with anti-CD3/CD28 Dynabeads was done at a cell:bead ratio of 1:1. A sufficient volume of beads was removed and washed in DPBS prior to direct addition to the cell suspension. Accordingly, stimulation culture was plated at 2mL YH5 media with 100 IU/mL IL-2 per well in a 24 well plate, as above, and incubated for 3 days.

Stimulation with soluble anti-CD3/CD28 was done by direct addition of CD3/CD28 ImmunoCult^{™} reagent at a concentration of 25 µl per mL (i.e. 50 µl per well of the 24-well plate as above) and incubated for 3 days.

Stimulation with EBV transformed lymphoblastoid B cell line (BLCL) was done at a ratio of 1 EBV antigen-specific T cell to four BLCL. Briefly, CD3+, EBV-antigen specific T cells were removed from liquid nitrogen storage, thawed, suspended in YH media, and allowed to recover. In preparation for stimulation culture, EBV-BLCL were irradiated with a total dose of 90Gy (9000rads) of radiation. EBV-BLCL antigen-specific T cells were combined in YH5 media with 100 IU/mL IL-2 in the same 24-well plate as above and incubated for 3 days.

### CAR transduction

In preparation of transduction, 24-well plates were prepared as follows: 0.5 mL of Retronectin (10µg/mL) was added to each well of a 24-well non-tissue culture-treated plate and incubated for 2 hours at room temperature. Retronectin was then removed and replaced with 0.5 mL blocking buffer and incubated at room temperature for 30 minutes. Blocking buffer was then removed and each well was washed with at least 1mL of wash buffer. Plates were kept with wash buffer at 4°C until ready for use.

Lentiviral vector encoding the CAR (comprising either a 4-1BB or CD28 signal transduction domain) was thawed at room temperature and added to each well of the Retronectin-coated plates to achieve an MOI (multiplicity of infection) or 15 viral particles/cell (see Table 2). The plates were then wrapped in paraffin film and centrifuged at 2000xg for 2 hours at 32°C, during which time cells from each stimulation group were counted and resuspended in YH5 media at a concentration of 0.5 × 10⁶ cells/mL. Following centrifugation, viral supernatant was aspirated from each well and replaced with 1mL of the prepared cell suspensions, including control wells. Plates were centrifuged at 1000xg for 15 minutes at 32°C and then incubated at 37°C, 5% CO2 overnight.

**Table 3: FACS reagents - conjugated antibodies and viability dyes**

| **Antibody/Reagent** | **Clone** | **Manufacturer** |
|---|---|---|
| Anti-CD3 APC-Cy7 | UCHT1 | Biolegend |
| CD19-Fc Biotin | | AcroBiosystems |
| Streptavidin PE | | eBioscience |
| Anti-CD4 BV421 | RPA-T4 | BD Biosciences |
| Anti-CD8 APC | SK1 | Biolegend |
| Live/Dead BV510 | | Tonbo |
| CD45RA PerCP-Cy5.5 | HI100 | Biolegend |
| CD45RO PE-Cy7 | UCHL1 | Biolegend |
| CD62L BB515 | DREG-56 | BD Biosciences |

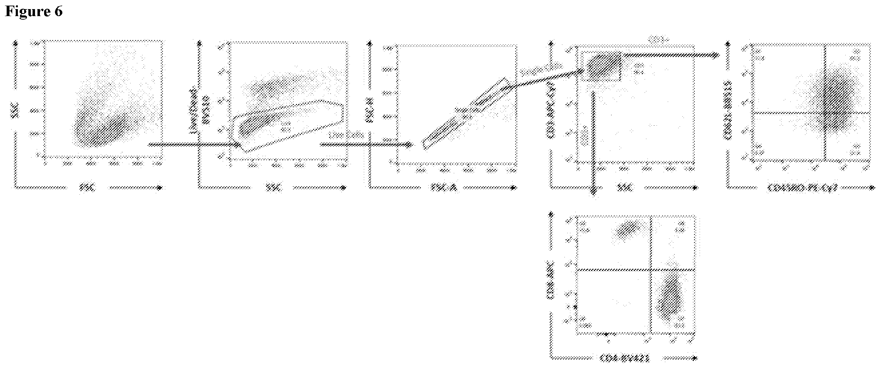

Cells from each stimulation group were pooled, counted, and resuspended at a concentration of 0.5-1.0×10⁶ cells/mL of fresh YH5 media before returning to incubation at 37°C, 5% CO₂ for 2 days. Pooling and resuspension was repeated every two days until the seventh day (harvest). Once harvested cells were labeled (see Table 3) and a flow cytometry gating strategy was applied to identify CD3, CD4, CD8, CD62L and CD45RO on the CAR-expressing T cells (see Figure 6). Briefly, cell signals of interest were first identified by size and granularity (i.e. forward scatter (FSC) vs. side scatter (SSC)). Living cells were then identified based on staining with a viability dye (*e*.*g*., Live/Dead^{™} BV510). Viable single cells were then gated based on pulse-area vs. pulse-height (FSC-A vs. FSC-H). Individual CD3⁺ T cells and subsequent subpopulations (*e*.*g*. CD4⁺, CD8⁺, central memory T cells) could then be identified using fluorescently-labeled antibodies.

### Results

**Table 4: Frequency of CD62L⁺/CD45RO⁺ on CD19-CAR-T cells**

| **Simulation Culture** | **Frequency of CD62L⁺/CD45RO⁺** | | **Frequency of CD62L⁻/CD45RO⁻** | |
|---|---|---|---|---|
| **Signal domain** | **4-1BBz** | **CD28z** | **4-1BBz** | **CD28z** |
| **BLCL** | 61.2% | 65.5% | 21.9% | 19.1% |
| **Soluble anti-CD3/CD28** | 36.3% | 35.1% | 31.8% | 29.9% |
| **Bead-bound anti-CD3/CD28** | 35.1% | 44.9% | 45% | 45.3% |
| **Unstimulated** | 25.2% | | 31.9% | |

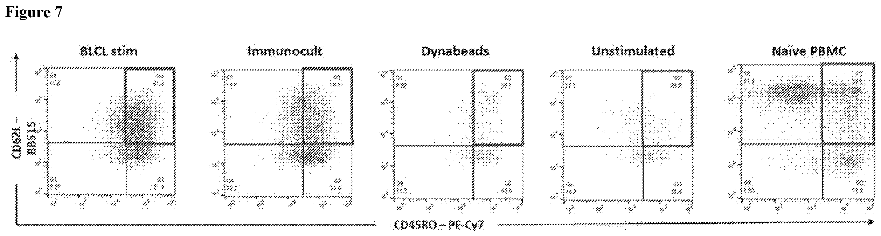

Flow cytometric evaluation indicated that transduced CD19-CAR-T cells stimulated with EBV-BLCLs showed higher percentages of central memory T cell subsets as evaluated by CD62L⁺/CD45RO⁺ cells compared to soluble CD3/CD28 or bead stimulation (see Figure 7). CD19-CAR-T cells stimulated with soluble or bead-bound CD3/CD28 had higher percentages of effector memory T cell subsets as evaluated by CD62L⁻/CD45RO⁺ cells compared to BLCL stimulated cells (see Table 4).

**Table 5: Frequency of CD4 and CD8 on CD19-CAR-T cells (gated on CD3)**

| **Stimulation** | **Frequency of CD3⁺CD4⁺** | | **Frequency of CD3⁺CD8⁺** | |
|---|---|---|---|---|
| | 4-1BBz | 28z | 4-1BBz | 28z |
| **BLCL** | 83.5 | 83.8 | 15.6 | 15.2 |
| **CD3/CD28 ImmunoCult** | 52.8 | 45.7 | 43.1 | 49.3 |
| **CD3/CD28 Dynabeads** | 29.5 | 22.3 | 56.5 | 53.0 |
| **Unstimulated** | 54.5 | | 44.1 | |

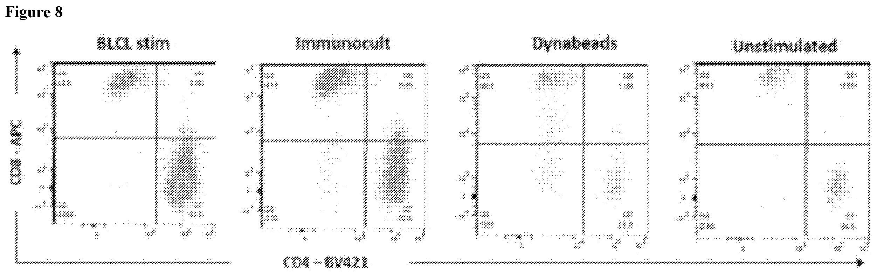

CD4 and CD8 expression was also evaluated on CD19-CAR-T cells. Of the CD3⁺ population of CD19-CAR-T cells, there was a higher percentage of CD4⁺ cells when stimulated with BLCLs compared to CD3/CD28 stimulation (see Figure 8 and Table 5).

These data demonstrate that stimulation of CAR-T cell cultures with BLCLs expressing CAR-targeted antigens leads to a shift to predominant central memory phenotype when compared to results from CD3/CD28 stimulation through bead-bound or soluble antibodies. This study strongly supports the potential for improved quality of CAR-T cell cultures that utilize antigen-postive APC stimulation (as represented by antigen-presenting BLCLs) versus the quality expected when using standard anti-CD3/CD28 bead or other acellular modes of stimulation.

### Example 3: anti-CD19-CAR-EBV-CTLs exert potent and specific cytotoxicity

Similarly as above, lentiviral vector encoding the CAR (comprising either a 4-1BB or CD28 signal transduction domain) was used for transduction of EBV-BLCL-stimulated, CD3⁺, antigen-specific T cells.

Briefly, CAR-expressing, virus-specific T cells were prepared in the following manner.
- Day 0, PBMC samples were thawed, and CD3⁺ cells were enriched (e.g., by live FACS or anti-CD3-coated magnetic beads). These CD3⁺ T cells were then stimulated by culturing with EBV-antigen-presenting BLCLs as described herein.
- 11 days post-stimulation (on Day 11), the culture was depleted of NK cells (e.g., using anti-CD56 beads) and re-stimulated with fresh EBV-antigen-presenting BLCLs at a responder/stimulator ratio of 1:4 for 7 days.
- On Day 18, the culture was stimulated a third time, at a responder/stimulator ratio of 4:1 for 2 days.
- On Day 20 transduction was initiated on with a viral vector encoding a chimeric antigen receptor (CAR) and returned to incubation in standard (YH media) culture to allow for expansion of the transduced, virus-specific T cells. Optionally, an NK cell depletion step may be employed immediately prior to transduction.
- By Day 25, CAR-expression was assessed (e.g., by FACS analysis) and CAR-expressing, virus-specific T cells (effectors) could be added to target cultures for cytotoxicity assays. Optionally, cells could be frozen at day 25, then thawed and tested for cytotoxicity at day 28.

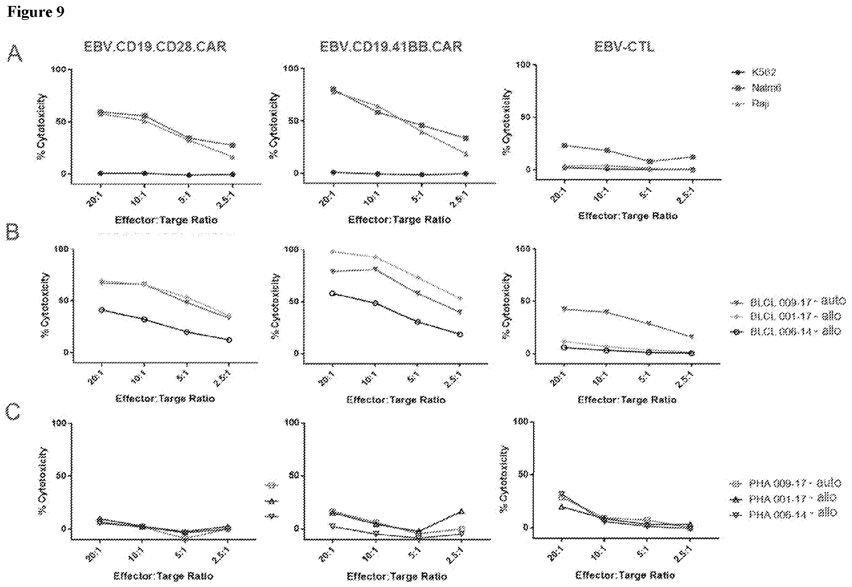

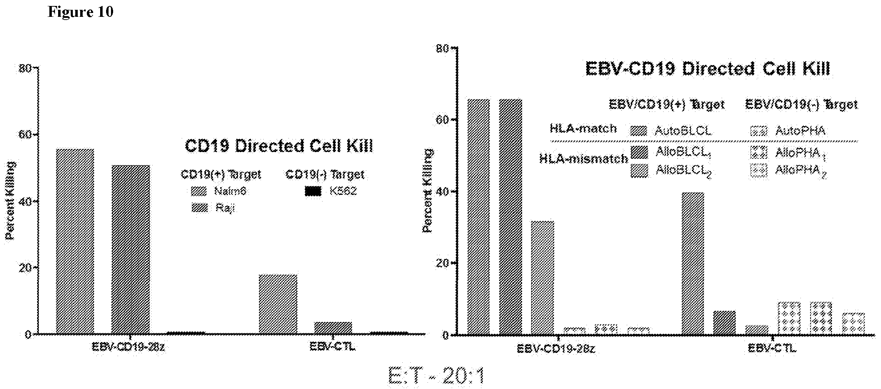

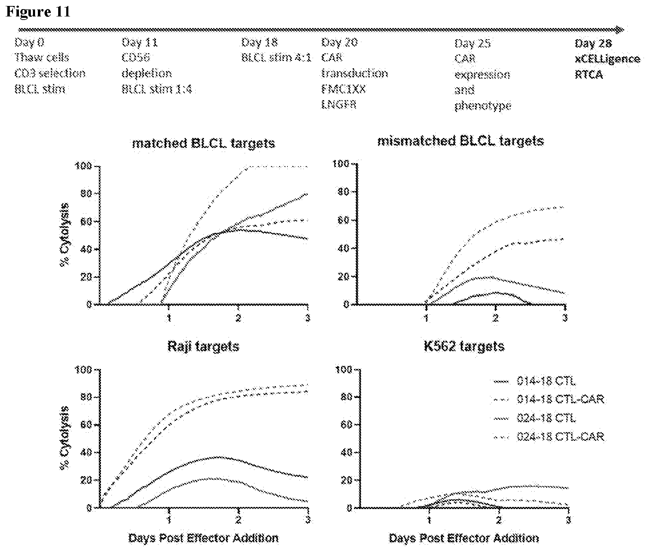

Cytotoxicity, measured by LDH release, could be observed after 4 hours in co-culture with EBV-CAR19-CAR T cells or control EBV CTLs at the indicated E:T ratios (see Figure 9 and 10). EBV-CD19-CAR T cells demonstrate HLA-independent, CD19-specific cytotoxicity with low allo-cytotoxicity as observed by the specific killing of CD19⁺ (NALM6 and Raji) cells and EBV⁺/CD19⁺ HLA-matched and mismatched BLCLs (see Figure 9, A and B; and Figure 10). In contrast, K562 cells, and HLA-matched and mismatched PHA blasts, all of which lack EBV and CD19 antigen, are not killed (See Figure 9, A and C; and Figure 10). Moreover, EBV-sensitized, anti-CD19 CAR T cells were cytotoxic to all CD19⁺ cell lines with less off-target cytoxicity, i.e., less or non-existent cytotoxicity to cells lacking both CD19 and EBV expression. When observed for three days following effector addition (i.e., EBV-CTL or EBV-CD19 CAR T cell addition) specific and potent HLA-independnet cytolysis was observed in targeted cells. Cytolysis was induced in both HLA-matched (BLCL targets) and HLA-mismatched (BLCL and Raji targets) cells. However, EBV-CTL could only induce significant cytolysis in matched BLCL target cells (see Figure 11).

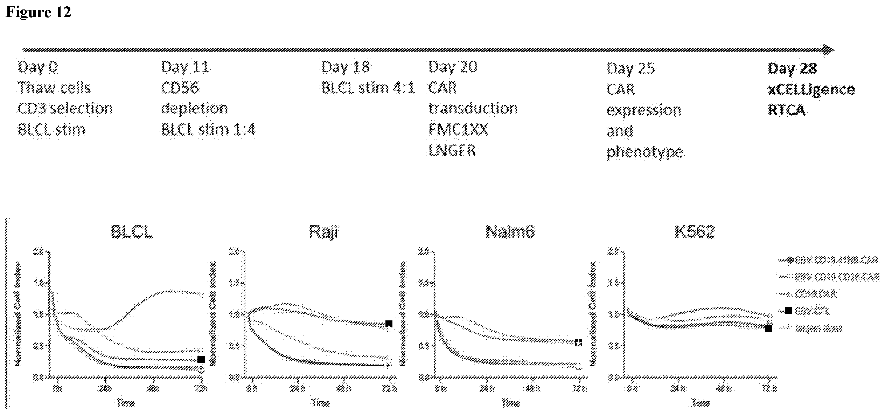

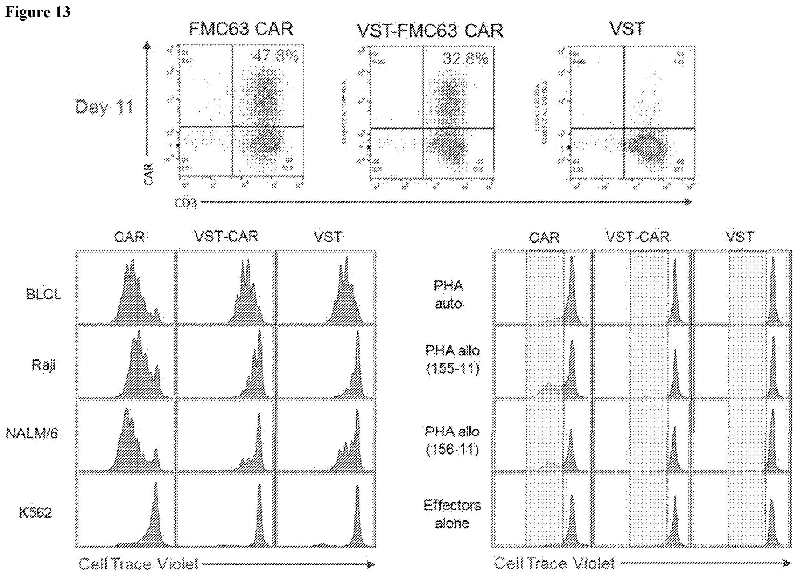

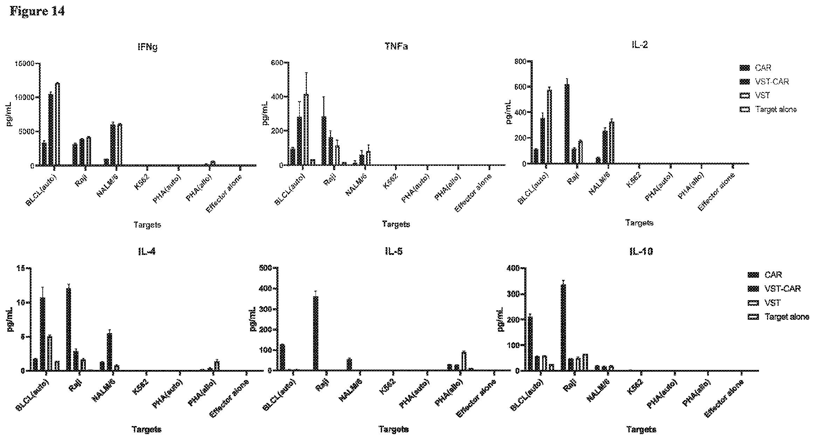

When compared to conventionally produced CAR T cells (i.e., without enrichment or antigen stimulation of starting T cultures), antigen-specific CAR T cells (e.g., anti-CD19-CD28-CAR-EBV-CTLs) exhibit comparable, if not better, cytotoxicity (see Figure 12). However, anti-CD19-CD28-CAR-EBV-CTLs appear to be less alloreactive. The proliferative capacity of EBV-specific CD19-CAR T cells was observed by CellTrace^{™} Violet dilution assays upon co-culture with the indicated cell lines (see Figure 13). Anti-CD19-CD28-CAR-EBV-CTLs retained the ability to kill B-lymphoblastoid cell lines (BLCL) (Figure 13, bottom-left) but spared autologous and allogeneic PHA-blast targets lacking CD19 and EBV antigen expression with notably less alloreactivity relative to conventional CAR T cells (Figure 13, bottom-right, shaded). What is more, relative to conventionally produced CAR T-cells, anti-CD19-CD28-CAR-EBV-CTLs exhibit a distinct cytokine profile that features increased IFNy production (see Figure 14).

## Claims

1. A method of generating a composition comprising cytotoxic T cells that are specific for binding to a first antigen and that express a chimeric antigen receptor (CAR) specific for binding to a second antigen, said method comprising:
i) preparing a culture of CD3⁺ T cells, wherein the culture of CD3⁺ T cells comprises a sample of cells comprising CD3⁺ T cells that has been obtained from a subject;
ii) stimulating said CD3⁺ T cells to recognize and bind to said first antigen through a T-cell receptor by co-culturing the culture of CD3⁺ T cells with lymphoblastoid B cells (BLCLs) that present said first antigen on their cell surface, thereby activating and inducing proliferation of CD3⁺ T cells being specific for said first antigen;
iii) transducing the CD3⁺ T cells of said co-culture with a viral vector comprising a nucleic acid sequence encoding a CAR that binds to said second antigen;
iv) culturing the CAR-transduced CD3⁺ T cells to allow proliferation of CAR-expressing, first-antigen-specific cytotoxic T cells; and
v) harvesting the CAR-expressing, first-antigen-specific cytotoxic T cells.

2. The method of claim 1, wherein the composition is an adoptive immunotherapy composition, wherein said adoptive immunotherapy composition is provided in step iv).

3. The method of claim 1 or 2, wherein the culture of CD3⁺ T cells comprises a sample of PBMCs comprising T CD3⁺ cells that has been obtained from a subject.

4. The method of any one of claims 1 to 3, further comprising incubating the culture of steps i), ii), iii), iv) or any combination thereof, with one or more cytokines, wherein the cytokine is preferably IL-2.

5. The method of any one of claims 1 to 4, wherein in said co-culturing of step (ii) the ratio of cells in the culture of CD3⁺ T cells to BLCLs is about 20: 1.

6. The method of any one of claims 1-5, wherein the co-culture of step (ii) is maintained for at least 2 days after antigen presentation by BLCLs prior to transduction with a CAR-encoding vector in step (iii).

7. The method of any one of claims 1-6, further comprising after step (ii) and before (iii) re-stimulating the culture of CD3⁺ T cells with BLCLs that present said first antigen on their cell surface.

8. The method of any one of claims 1-7, further comprising stimulating said transduced CD3⁺ T cells of step iii) with BLCLs that present said first antigen on their cell surface.

9. The method of claim 7 and 8, wherein CD56⁺ cells are depleted from the culture immediately prior to stimulating them with said BLCLs.

10. The method of any one of claims 1 to 9, wherein in said co-culturing of step (ii) the ratio of cells in the culture of CD3⁺ T cells to BLCLs is about 0.2: 1 to about 20: 1.

11. The method of any one of claims 1 to 10, wherein said prepared culture of CD3⁺ T cells stimulated to recognize and bind to said first antigen through a T-cell receptor is re-cultured with BLCLs that present said first antigen on their surface prior to CAR transduction.

12. The method of claim 11, wherein a first re-culture step is initiated at least 7 days after initiation of the co-culture step.

13. The method of any one of claims 1 to 12, wherein the culture of CD3⁺ T cells and the BLCLs are derived from the same donor.

14. The method of any one of claims 1 to 13, wherein the BLCLs are gamma irradiated antigen-presenting stimulator cells.

15. The method of any one of claims 1 to 14, wherein the BLCLs are infected with a native and/or wild-type virus comprising at least one immunogenic peptide antigen comprising a T cell epitope, wherein said least one immunogenic peptide antigen is the first antigen.

16. The method of claim 15, wherein said at least one immunogenic peptide antigen is an Epstein-Bar virus (EBV), cytomegalovirus (CMV), human papilloma virus (HPV), BK virus (BKV), John-Cunningham virus (JCV), Merkel cell virus (MCV), human T-lymphotropic virus (HTLV) or human immunodeficiency virus (HIV) antigen.

17. The method of claim 15 or 16, wherein said at least one immunogenic peptide antigen comprises an EBV LMP1 peptide or fragment thereof, an EBV LMP2A peptide or fragment thereof, or an EBV EBNA 1 peptide or fragment thereof.

18. The method of any one of claims 1 to 17, further comprising freezing and storing the harvested CAR-expressing, first-antigen-specific cytotoxic T cells for administering to a patient in need at a future date.

19. The method of any one of claims 1 to 18, wherein the viral vector comprising the nucleic acid sequence encoding the CAR is a lentiviral or retroviral vector, preferably a replication incompetent lentiviral or retroviral vector.

20. The method of any one of claims 1 to 19, wherein the CAR comprises one or more signal transduction domains, wherein the one or more signal transduction domains preferably comprise a CD28 signaling domain, a 4-1 BB signaling domain, a CD3 signaling domain, or variants or fragments thereof.

21. The method of claim 20, wherein the CAR comprises (i) a variant CD3ζ domain lacking at least one functional ITAM region, or (ii) a Mut06 domain.

## Patentansprüche

1. Verfahren zur Erzeugung einer Zusammensetzung, die zytotoxische T-Zellen umfasst, die spezifisch für die Bindung an ein erstes Antigen sind und die einen chimären Antigenrezeptor (CAR) exprimieren, der spezifisch für die Bindung an ein zweites Antigen ist, wobei das Verfahren umfasst:
i) Herstellen einer Kultur von CD3⁺-T-Zellen, wobei die Kultur von CD3⁺-T-Zellen eine Zellprobe umfasst, die CD3⁺ T-Zellen umfasst und von einem Individuum erhalten wurde;
ii) Stimulierung der CD3⁺-T-Zellen zur Erkennung und Bindung an das erste Antigen über einen T-Zell-Rezeptor durch Co-Kultivierung der Kultur von CD3⁺-T-Zellen mit lymphoblastoiden B-Zellen (BLCLs), die das erste Antigen auf ihrer Zelloberfläche präsentieren und dadurch aktivieren und Induzieren der Proliferation von CD3⁺-T-Zellen, die spezifisch für das erste Antigen sind;
iii) Transduzieren der CD3⁺-T-Zellen der Co-Kultur mit einem viralen Vektor, der eine Nukleinsäuresequenz umfasst, die für einen CAR kodiert, der an das zweite Antigen bindet;
iv) Kultivierung der CAR-transduzierten CD3⁺-T-Zellen, um die Proliferation von CAR-exprimierenden, erstes Antigen-spezifischen zytotoxischen T-Zellen zu ermöglichen; und
v) Ernten der CAR-exprimierenden, erstes Antigen-spezifischen zytotoxischen T-Zellen.

2. Verfahren nach Anspruch 1, wobei die Zusammensetzung eine adoptive Immuntherapie-Zusammensetzung ist, wobei die adoptive Immuntherapie-Zusammensetzung in Schritt iv) bereitgestellt wird.

3. Verfahren nach Anspruch 1 oder 2, wobei die Kultur von CD3⁺-T-Zellen eine Probe von PBMCs umfasst, die T-CD3⁺-Zellen umfasst und von einem Individuum erhalten wurde.

4. Verfahren nach einem der Ansprüche 1 bis 3, das außerdem das Inkubieren der Kultur der Schritte i), ii), iii), iv) oder einer beliebigen Kombination davon mit einem oder mehreren Zytokinen umfasst, wobei das Zytokin vorzugsweise IL-2 ist.

5. Verfahren nach einem der Ansprüche 1 bis 4, wobei in der Co-Kultivierung von Schritt (ii) das Verhältnis von Zellen in der Kultur von CD3⁺-T-Zellen zu BLCLs etwa 20:1 beträgt.

6. Verfahren nach einem der Ansprüche 1 bis 5, wobei die Co-Kultur von Schritt (ii) nach der Antigenpräsentation durch BLCLs vor der Transduktion mit einem CAR-kodierenden Vektor in Schritt (iii) für mindestens 2 Tage aufrechterhalten wird.

7. Verfahren nach einem der Ansprüche 1 bis 6, das nach Schritt (ii) und vor (iii) außerdem die erneute Stimulierung der Kultur von CD3⁺-T-Zellen mit BLCLs umfasst, die das erste Antigen auf ihrer Zelloberfläche präsentieren.

8. Verfahren nach einem der Ansprüche 1 bis 7, das weiterhin die Stimulierung der transduzierten CD3⁺-T-Zellen aus Schritt iii) mit BLCLs umfasst, die das erste Antigen auf ihrer Zelloberfläche präsentieren.

9. Verfahren nach Anspruch 7 und 8, wobei CD56⁺-Zellen aus der Kultur abgereichert werden unmittelbar vor ihrer Stimulierung mit den BLCLs.

10. Verfahren nach einem der Ansprüche 1 bis 9, wobei in der Co-Kultivierung aus Schritt (ii) das Verhältnis von Zellen in der Kultur von CD3⁺-T-Zellen zu BLCLs etwa 0,2:1 bis etwa 20:1 beträgt.

11. Verfahren nach einem der Ansprüche 1 bis 10, wobei die vorbereitete Kultur von CD3⁺-T-Zellen, die über einen T-Zell-Rezeptor zur Erkennung und Bindung des ersten Antigens stimuliert werden, mit BLCLs neu kultiviert wird, die das erste Antigen auf ihrer Oberfläche präsentieren vor der CAR-Transduktion.

12. Verfahren nach Anspruch 11, wobei ein erster Rekultivierungsschritt mindestens 7 Tage nach Beginn des Co-Kultivierungsschritts eingeleitet wird.

13. Verfahren nach einem der Ansprüche 1 bis 12, wobei die Kultur der CD3⁺-T-Zellen und die BLCLs vom selben Spender stammen.

14. Verfahren nach einem der Ansprüche 1 bis 13, wobei die BLCLs gammabestrahlte Antigen-präsentierende Stimulatorzellen sind.

15. Verfahren nach einem der Ansprüche 1 bis 14, wobei die BLCLs mit einem nativen und/oder Wildtyp-Virus infiziert werden, der mindestens ein immunogenes Peptidantigen umfasst, das ein T-Zell-Epitop umfasst, wobei das mindestens eine immunogene Peptidantigen das erste Antigen ist.

16. Verfahren nach Anspruch 15, wobei das mindestens eine immunogene Peptidantigen ein Epstein-Bar-Virus (EBV)-, Cytomegalovirus (CMV)-, humanes Papillomavirus (HPV)-, BK-Virus (BKV)-, John-Cunningham-Virus (JCV)-, Merkelzellvirus (MCV)-, humanes T-lymphotropes Virus (HTLV)- oder humanes Immundefizienzvirus (HIV)-Antigen ist.

17. Verfahren nach Anspruch 15 oder 16, wobei das mindestens eine immunogene Peptidantigen ein EBV-LMP1-Peptid oder ein Fragment davon, ein EBV-LMP2A-Peptid oder ein Fragment davon oder ein EBV-EBNA-1-Peptid oder ein Fragment davon umfasst.

18. Verfahren nach einem der Ansprüche 1 bis 17, das weiterhin das Einfrieren und Lagern der geernteten CAR-exprimierenden, erstes Antigen-spezifischen zytotoxischen T-Zellen zur Verabreichung an einen bedürftigen Patienten zu einem zukünftigen Zeitpunkt umfasst.

19. Verfahren nach einem der Ansprüche 1 bis 18, wobei der virale Vektor, der die für das CAR kodierende Nukleinsäuresequenz umfasst, ein lentiviraler oder retroviraler Vektor ist, vorzugsweise ein replikationsinkompetenter lentiviraler oder retroviraler Vektor.

20. Verfahren nach einem der Ansprüche 1 bis 19, wobei der CAR eine oder mehrere Signaltransduktionsdomänen umfasst, wobei die eine oder mehreren Signaltransduktionsdomänen vorzugsweise eine CD28-Signalisierungsdomäne, eine 4-1 BB-Signalisierungsdomäne, eine CD3-Signalisierungsdomäne oder Varianten oder Fragmente davon umfassen.

21. Verfahren nach Anspruch 20, wobei der CAR (i) eine CD3ζ-Variantendomäne, der mindestens eine funktionelle ITAM-Region fehlt, oder (ii) eine Mut06-Domäne umfasst.

## Revendications

1. Méthode de génération d'une composition comprenant des lymphocytes T cytotoxiques qui sont spécifiques pour la liaison à un premier antigène et qui expriment un récepteur d'antigène chimérique (CAR) spécifique pour la liaison à un deuxième antigène, ladite méthode comprenant :
i) la préparation d'un culture de lymphocytes T CD3⁺, où la culture des lymphocytes T CD3⁺ comprend un échantillon de cellules comprenant des lymphocytes T CD3⁺ qui a été obtenu auprès d'un sujet ;
ii) la stimulation desdits lymphocytes T CD3⁺ pour qu'ils identifient et se lient audit premier antigène par le biais d'un récepteur de lymphocyte T par co-culture de la culture des lymphocytes T CD3⁺ avec des cellules B lymphoblastoïdes (BLCL) qui présentent ledit premier antigène sur leur surface cellulaire, ce qui permet d'activer et d'induire la prolifération des lymphocytes T CD3⁺ qui sont spécifiques pour ledit premier antigène ;
iii) la transduction des lymphocytes T CD3⁺ de ladite co-culture avec un vecteur viral comprenant une séquence d'acide nucléique codant pour un CAR qui se lie audit deuxième antigène ;
(iv) la culture des lymphocytes T CD3⁺ transduit par un CAR pour permettre la prolifération des lymphocytes T cytotoxiques spécifique au premier antigène, exprimant un CAR ; et
(v) la récolte des lymphocytes T cytotoxiques spécifiques au premier antigène, exprimant un CAR.

2. Méthode selon la revendication 1, dans laquelle la composition est une composition d'immunothérapie adoptive, dans laquelle ladite composition d'immunothérapie adoptive est fournie à l'étape iv).

3. Méthode selon la revendication 1 ou 2, dans laquelle la culture des lymphocytes T CD3⁺ comprend un échantillon de PBMC comprenant des lymphocytes T CD3⁺ qui a été obtenu auprès d'un sujet.

4. Méthode selon l'une quelconque des revendications 1 à 3, comprenant en outre l'incubation de la culture des étapes i), ii), iii), iv) ou toute combinaison de celles-ci, avec une ou plusieurs cytokines, où la cytokine est de préférence IL-2.

5. Méthode selon l'une quelconque des revendications 1 à 4, dans laquelle, dans ladite co-culture de l'étape (ii), le rapport des cellules dans la culture des lymphocytes T CD3⁺ sur les BLCL est d'environ 20 : 1.

6. Méthode selon l'une quelconque des revendications 1 à 5, dans laquelle, ladite co-culture de l'étape (ii) est maintenue pendant au moins 2 jours après la présentation d'antigène par les BLCL avant la transduction avec un vecteur codant pour un CAR à l'étape (iii).

7. Méthode selon l'une quelconque des revendications 1 à 6, comprenant en outre, après l'étape (ii) et avant (iii) la restimulation de la culture des lymphocytes T CD3⁺ avec les BLCL qui présentent ledit premier antigène sur leur surface cellulaire.

8. Méthode selon l'une quelconque des revendications 1 à 7, comprenant en outre la stimulation desdits lymphocytes T CD3⁺ transduits de l'étape iii) avec les BLCL qui présentent ledit premier antigène sur leur surface cellulaire.

9. Méthode selon la revendication 7 ou 8, dans laquelle les cellules CD56⁺ sont déplétées de la culture en immédiatement avant leur stimulation avec lesdites BLCL.

10. Méthode selon l'une quelconque des revendications 1 à 9, dans laquelle, dans ladite co-culture de l'étape (ii), le rapport des cellules dans la culture des lymphocytes T CD3⁺ sur les BLCL est d'environ 0,2 : 1 à environ 20 : 1.

11. Méthode selon l'une quelconque des revendications 1 à 10, dans laquelle ladite culture préparée des lymphocytes T CD3⁺ stimulés pour identifier et se lier audit premier antigène par le biais d'un récepteur de lymphocyte T est re-cultivée avec les BLCL qui présentent ledit premier antigène sur leur surface avant la transduction de CAR.

12. Méthode selon la revendication 11, dans laquelle une première étape de re-culture est initiée au moins 7 jours après l'initiation de l'étape de co-culture.

13. Méthode selon l'une quelconque des revendications 1 à 12, dans laquelle la culture de lymphocytes T CD3⁺ et les BLCL sont dérivées du même donneur.

14. Méthode selon l'une quelconque des revendications 1 à 13, dans laquelle les BLCL sont des cellules stimulantes présentatrices d'antigène gamma-irradiées.

15. Méthode selon l'une quelconque des revendications 1 à 14, dans laquelle les BLCL sont infectées par un virus natif et/ou de type sauvage, comprenant au moins un antigène de peptide immunogène comprenant un épitope de lymphocyte T, dans laquelle ledit au moins un antigène de peptide immunogène est le premier antigène.

16. Méthode selon la revendication 15, dans laquelle ledit au moins un antigène de peptide immunogène est un antigène de virus Epstein-Bar (EBV), cytomégalovirus (CMV), virus du papillome humain (HPV), virus BK (BKV), virus John-Cunningham (JCV), polyomavirus à cellules de Merkel (MCV), virus T-lymphotropique humain (HTLV) ou virus de l'immunodéficience humaine (VIH).

17. Méthode selon la revendication 15 ou 16, dans laquelle ledit au moins un antigène de peptide immunogène comprend un peptide de EBV LMP1 ou un fragment de celui-ci, un peptide de EBV LMP2A ou un fragment de celui-ci, ou un peptide de EBV EBNA 1 ou un fragment de celui-ci.

18. Méthode selon l'une quelconque des revendications 1 à 17, comprenant en outre la congélation et le stockage des lymphocytes T cytotoxiques récoltés spécifiques au premier antigène, exprimant un CAR pour l'administration à un patient en ayant besoin à une date future.

19. Méthode selon l'une quelconque des revendications 1 à 18, dans laquelle le vecteur viral comprenant la séquence d'acide nucléique codant pour le CAR est un vecteur lentiviral ou rétroviral, de préférence un vecteur lentiviral ou rétroviral incompétent en réplication.

20. Méthode selon l'une quelconque des revendications 1 à 19, dans laquelle le CAR comprend un ou plusieurs domaines de transduction de signaux, dans laquelle les un ou plusieurs domaines de transduction de signaux comprennent de préférence un domaine de signalisation CD28, un domaine de signalisation 4-1 BB, un domaine de signalisation CD3, ou des variants ou fragments de ceux-ci.

21. Méthode selon la revendication 20, dans laquelle le CAR comprend (i) un domaine CD3ζ variant dépourvu d'au moins une région ITAM fonctionnelle, ou (ii) un domaine Mut06.
